# EUROPEAN PATENT SPECIFICATION

(11) **EP 3 090 059 B1**
(45) Date of publication and mention of the grant of the patent: **06.06.2018**
(21) Application number: 14827245.3
(22) Date of filing: 31.12.2014
(51) Int. Cl.: C12Q 1/68

(54) **LUNG CANCER DETERMINATIONS USING MIRNA RATIOS**
MICRO-RNA QUOTIENTEN ZUR BESTIMMUNG VON LUNGENKREBS
DIAGNOSTIC DU CANCER DU POUMON EN UTILISANT LE RAPPORT MIARN

(30) Priority: 05.01.2014 US 201461923758 P; 12.01.2014 US 201461926323 P
(43) Date of publication of application: 09.11.2016
(73) Proprietor: Biomirna Holdings Ltd., Dublin 4 (IE)
(72) Inventor: SOZZI, Gabriella, I-20122 Milan (IT); BOERI, Mattia, I-20122 Milan (IT); PASTORINO, Ugo, I-20123 Milan (IT)
(74) Representative: Cooley (UK) LLP
(86) International application number: PCT/EP2014/079499
(87) International publication number: WO 2015/101653

(56) References cited:
- WO-A1-2009/070653
- WO-A2-2011/053257
- WO-A2-2012/107841
- M. BOERI ET AL: "MicroRNA signatures in tissues and plasma predict development and prognosis of computed tomography detected lung cancer", PROCEEDINGS OF THE NATIONAL ACADEMY OF SCIENCES, vol. 108, no. 9, 1 March 2011 (2011-03-01) , pages 3713-3718, XP055044109, ISSN: 0027-8424, DOI: 10.1073/pnas.1100048108
- QIAN S ET AL: "MicroRNA expression profile of bronchioalveolar stem cells from mouse lung", BIOCHEMICAL AND BIOPHYSICAL RESEARCH COMMUNICATIONS, ACADEMIC PRESS INC. ORLANDO, FL, US, vol. 377, no. 2, 12 December 2008 (2008-12-12), pages 668-673, XP025646136, ISSN: 0006-291X, DOI: 10.1016/J.BBRC.2008.10.052 [retrieved on 2008-10-21]
- BOERI M ET AL: "Role of MicroRNAs in lung cancer: MicroRNA signatures in cancer prognosis", CANCER JOURNAL (UNITED STATES) 2012 LIPPINCOTT WILLIAMS AND WILKINS USA, vol. 18, no. 3, May 2012 (2012-05), pages 268-274, XP008175873, ISSN: 1528-9117
- JI QI ET AL: "MicroRNAs and lung cancers: from pathogenesis to clinical implications", FRONTIERS OF MEDICINE, SP HIGHER EDUCATION PRESS, HEIDELBERG, vol. 6, no. 2, 18 April 2012 (2012-04-18), pages 134-155, XP035065369, ISSN: 2095-0225, DOI: 10.1007/S11684-012-0188-4

## Description

### FIELD OF THE INVENTION

The present application generally relates to diagnosis and determining risk of lung cancer. More specifically, the application is directed to the use of miRNA expression ratios to determine a risk for manifesting a pulmonary tumor or an aggressive pulmonary tumor, and for determining the presence of a pulmonary tumor or an aggressive pulmonary tumor.

### BACKGROUND OF THE INVENTION

Lung cancer is the leading cause of cancer death worldwide (Jemal et al., CA Cancer J Clin, 61:69-9, 2011). Currently the majority of lung cancers are detected at an advanced stage where treatments have limited efficacy and survival rates are low. Detection of lung cancer at an early stage may significantly reduce mortality.

European randomized lung cancer screening trials, with an observational control arm but limited size, have not demonstrated mortality reductions to-date (Infante et al., Am J Respir Crit Care Med 180:445-453, 2009; Saghir et al., Thorax 67:296-301, 2012; Pastorino et al., Eur J Cancer Prev 21:308-315, 2012); however results from the large NCI-sponsored National Lung Screening Trial (NSLT) showed a 20% reduction in mortality with low-dose computed tomography (LDCT) screening of high risk individuals with a history of ≥30 pack-years and ≤15 years since quit smoking, compared to annual chest radiography (Aberle et al., N Engl J Med 365:395-409, 2011). The high false positive rates, the cost of screening the large number of individuals at high risk (in US estimated at $3.5 million), and the potential harms associated with LDCT screening remain considerations in clinical settings (Aberle et al., 2011; Goulart et al., J Natl Compr Canc Netw 10:267-275, 2012).

Thus, there is a need for additional methodologies to improve prediction, prognosis and diagnosis of lung cancer. The present invention addresses that need.

### SUMMARY OF THE INVENTION

The present invention is based in part on the discovery that assay methods may utilize circulating miRNA biomarkers to provide a three-level classifier for an overall "risk of developing or having a pulmonary tumor" assay.

Thus, in some embodiments, a method of determining the presence of a pulmonary tumor in a subject is provided. The method comprises:
(a) determine the expression ratio of an miRNA pair in a biological sample from the subject;
(b) compare the expression ratio from step (a) with a cut-off value determined from the average ratio of a plurality of corresponding miRNA pairs from a plurality of control samples;
(c) assign a positive score for the expression ratio of the miRNA pair in step (a) if the ratio exceeds the cut-off value in step (b), or assign a non-positive score for the expression ratio of the miRNA pair in step (a) if the ratio does not exceed the cut-off value in step (b);
(d) repeat steps (a) through (c) for additional miRNA pairs until either (1) at least nine miRNA pair expression ratios are assigned a positive score, or (2) after the comparison of the expression ratios of 27 miRNA pairs less than nine miRNA pair expression ratios are assigned a positive score,
   wherein the miRNA pairs comprise 106a/140-5p, 106a/142-3p, 126/140-5p, 126/142-3p, 133a/142-3p, 140-5p/17, 142-3p/148a, 142-3p/15b, 142-3p/17, 142-3p/21, 142-3p/221, 142-3p/30b, or 320/660, or the inverse ratios thereof; and
(e) categorize the presence of a pulmonary tumor as (i) positive if at least nine of the miRNA pair expression ratios are assigned a positive score, or (ii) negative if less than nine miRNA pair expression ratios are assigned a positive score.

According to another embodiment, a method of determining the presence of an aggressive pulmonary tumor in a subject comprises:
(a) determine the expression ratio of an miRNA pair in a biological sample from the subject;
(b) compare the expression ratio from step (a) with a cut-off value determined from the average ratio of a plurality of corresponding miRNA pairs from a plurality of control samples;
(c) assign a positive score for the expression ratio of the miRNA pair in step (a) if the ratio exceeds the cut-off value in step (b), or assign a non-positive score for the expression ratio of the miRNA pair in step (a) if the ratio does not exceed the cut-off value in step (b);
(d) repeat steps (a) through (c) for additional miRNA pairs until either (1) at least fourteen miRNA pair expression ratios are assigned a positive score, or (2) after the comparison of the expression ratios of 28 miRNA pairs less than fourteen miRNA pair expression ratios are assigned a positive score,
   wherein the miRNA pairs comprise 106a/16, 106/660, 16/17, 16/320, 17/660, 197/30b, 197/30c, 320/451, 320/486-5p, or 320/660, or the inverse ratios thereof; and
(e) categorize the presence of an aggressive pulmonary tumor as (i) positive if at least fourteen of the miRNA pair expression ratios are assigned a positive score, or (ii) negative if less than fourteen miRNA pair expression ratios are assigned a positive score.

According to yet another embodiment, a method of determining the risk of manifesting a pulmonary tumor in a subject comprises:
(a) determine the expression ratio of an miRNA pair in a biological sample from the subject;
(b) compare the expression ratio from step (a) with a cut-off value determined from the average ratio of a plurality of corresponding miRNA pairs from a plurality of control samples;
(c) assign a positive score for the expression ratio of the miRNA pair in step (a) if the ratio exceeds the cut-off value in step (b), or assign a non-positive score for the expression ratio of the miRNA pair in step (a) if the ratio does not exceed the cut-off value in step (b);
(d) repeat steps (a) through (c) for additional miRNA pairs until either (1) at least ten miRNA pair expression ratios are assigned a positive score, or (2) after the comparison of the expression ratios of 27 miRNA pairs, less than ten miRNA pair expression ratios are assigned a positive score,
   wherein the miRNA pairs comprise 133a/92a, 15b/21, 15b/30b, 15b/30c, 16/197, or 28-3p/451, or the inverse ratios thereof; and
(e) categorize the risk of manifesting a pulmonary tumor as (i) positive if at least ten miRNA pair expression ratios are assigned a positive score, or (ii) negative if less than ten miRNA expression ratios are assigned a positive score.

According to other embodiments, a method of determining the risk of manifesting an aggressive pulmonary tumor comprises:
(a) determine the expression ratio of an miRNA pair in a biological sample from the subject;
(b) compare the expression ratio from step (a) with a cut-off value determined from the average ratio of a plurality of corresponding miRNA pairs from a plurality of control samples;
(c) assign a positive score for the expression ratio of the miRNA pair in step (a) if the ratio exceeds the cut-off value in step (b), or assign a non-positive score for the expression ratio of the miRNA pair in step (a) if the ratio does not exceed the cut-off value in step (b);
(d) repeat steps (a) through (c) for additional miRNA pairs until either (1) at least fourteen miRNA pair expression ratios are assigned a positive score, or (2) after the comparison of the expression ratios of 28 miRNA pairs less than fourteen miRNA pair expression ratios are assigned a positive score,
   wherein the miRNA pairs comprise 106a/142-3p, 126/142-3p, 126/21, 126/92a, 142-3p/17, 142-3p/197, 142-3p/28-3p, 197/19b, 197/660, or 28-3p/660, or the inverse ratios thereof; and
(e) categorize the risk of manifesting an aggressive pulmonary tumor as (i) positive if at least fourteen miRNA pair expression ratios are assigned a positive score, or (ii) negative if less than fourteen miRNA pair expression ratios are assigned a positive score.

In other embodiments, a method for predicting the risk of developing or having a pulmonary tumor in a subject comprises:
(a) determining the presence of a pulmonary tumor signature described above;
(b) determining the presence of an aggressive pulmonary tumor signature described above;
(c) determining the risk of manifesting a pulmonary tumor signature described above;
(d) determining the risk of manifesting an aggressive pulmonary tumor signature described above;
(e) categorizing the subject as low risk of developing or having a pulmonary tumor if none of the signatures of steps (a)-(d) are categorized as positive;
(f) categorizing the subject as having an intermediate risk of developing or having a pulmonary tumor if at least the presence of a pulmonary tumor signature of step (a) is positive, or the risk of manifesting a pulmonary tumor signature of step (c) is positive; and both the presence of an aggressive pulmonary tumor signature of step (b) and the risk of manifesting an aggressive pulmonary tumor signature of step (d) are negative; and
(g) categorizing the subject as high risk of developing or having a pulmonary tumor if at least the presence of an aggressive pulmonary tumor signature of step (b) is positive or the risk of manifesting an aggressive pulmonary tumor signature of step (d) is positive.

A method of establishing treatment options for a subject is additionally provided. The method comprises testing the subject using any of the above methods, and determining treatment options according to the results of the method. The method may further comprise administering a treatment to the subject in need of such treatment.

While the disclosure has been described in conjunction with the detailed description thereof, the foregoing description is intended to illustrate and not limit the scope of the disclosure, which is defined by the scope of the appended claims. Other aspects, advantages, and modifications are within the scope of the following claims.

### BRIEF DESCRIPTION OF THE DRAWINGS

**FIG. 1** is a diagram describing characteristics of the Sample Consort in Example 1, The Multicentric Italian Lung Detection (MILD) study, 2005-2012.
**FIG. 2** is a graph and table showing three-year survival from date of blood sample collection according to miRNA signature classifier (MSC) among all subjects.

### DETAILED DESCRIPTION OF THE INVENTION

According to one embodiment, the present invention utilizes multiple ratios of miRNA expression levels to determine (a) the risk of manifesting a pulmonary tumor, (b) the risk of manifesting an aggressive pulmonary tumor, (c) the presence of a pulmonary tumor, (d) the presence of an aggressive tumor, and, when combining the risk detected in steps (a)-(d), determining the overall risk of having or developing a pulmonary tumor.

The present invention may include elimination of samples having detectable levels of hemolysis, e.g., by including the use of a miRNA hemolysis classifier; use of a three-level (Low, Intermediate or High) "risk of disease" classifier instead of a two-level (Low, High) classifier; or the use of control plasma samples from single subjects, instead of pools. Three miRNAs may be included, miR-101, miR-145 and miR-133a, which were excluded in the validation step of previous assays because of high variability in the control pools.

According to an exemplary method of the present invention, the "microRNA signature classifier" (MSC) provides a screening sensitivity of 87% for MSC alone and 98% when combined with low-dose computed tomography (LDCT) screening Accordingly, the MSC can be used separately or in combination with other methods, such as LDCT, in a synergistic approach to improve the effectiveness of LDCT for lung cancer screening by avoiding further rounds of LDCTs in a large proportion of subjects and unnecessary invasive diagnostic follow-up. The prognostic and diagnostic performance of MSC alone, as detailed in this application, demonstrates that independent of tumor stage, the miRNAs measured within the MSC are not simply an output of tumor load, but rather indicators of pathogenesis related to tumor aggressiveness.

The development of MSC was based on a non-biased computational approach of screening 4,950 ratios of 100 different plasma miRNAs for the selection of the optimal set of miRNA ratios for lung cancer detection and association with poor prognosis (Boeri et al., Proc Natl Acad Sci USA 108:3713-3718, 2011). These miRNA ratios may reflect regulation between competing mechanisms of miRNA regulation of miRNAs within different cellular components of the tumor and the surrounding microenvironment. Without being bound by any particular theory, stromal cells may be activated by the inflamed lung microenvironment, releasing specific miRNAs into the circulation that could be functionally engaged in the regulation of target genes associated with neoplastic transformation.

The MSC utilizes a robust assay of plasma-derived miRNA signatures. The MSC has diagnostic performance for malignant disease presence, risk of future malignancy and ability to distinguish lung cancers from the large majority of benign LDCT-detected pulmonary nodules. The particular signatures which may be utilized by the MSC are detailed herein.

### "Presence of Pulmonary Tumor" Signature

In some embodiments, a method of determining the presence of a pulmonary tumor in a subject is provided. The method comprises:
(a) determine the expression ratio of an miRNA pair in a biological sample from the subject;
(b) compare the expression ratio from step (a) with a cut-off value determined from the average ratio of a plurality of corresponding miRNA pairs from a plurality of control samples;
(c) assign a positive score for the expression ratio of the miRNA pair in step (a) if the ratio exceeds the cut-off value in step (b), or assign a non-positive score for the expression ratio of the miRNA pair in step (a) if the ratio does not exceed the cut-off value in step (b);
(d) repeat steps (a) through (c) for additional miRNA pairs until either (1) at least nine miRNA pair expression ratios are assigned a positive score, or (2) after the comparison of the expression ratios of 27 miRNA pairs less than nine miRNA pair expression ratios are assigned a positive score,
   wherein the miRNA pairs comprise 106a/140-5p, 106a/142-3p, 126/140-5p, 126/142-3p, 133a/142-3p, 140-5p/17, 142-3p/148a, 142-3p/15b, 142-3p/17, 142-3p/21, 142-3p/221, 142-3p/30b, or 320/660, or the inverse ratios thereof; and
(e) categorize the presence of a pulmonary tumor as (i) positive if at least nine of the miRNA pair expression ratios are assigned a positive score, or (ii) negative if less than nine miRNA pair expression ratios are assigned a positive score.

In some embodiments, the miRNA pairs of this assay comprise more than 2, more than 3, more than 4, more than 5, more than 6, more than 7, more than 8, more than 9, more than 10, more than 11, more than 12, or each of the ratios 106a/140-5p, 106a/142-3p, 126/140-5p, 126/142-3p, 133a/142-3p, 140-5p/17, 142-3p/148a, 142-3p/15b, 142-3p/17, 142-3p/21, 142-3p/221, 142-3p/30b, and 320/660, or the inverse ratios thereof.

In other embodiments, the miRNA pairs can further comprise the ratios 106a/660, 106a/92a, 126/660, 140-5p/197, 140-5p/28-3p, 142-3p/145, 142-3p/197, 142-3p/28-3p, 17/660, 17/92a, 197/660, 197/92a, 19b/660, or 28-3p/660, or the inverse ratios thereof.

In still other embodiments, the miRNA pairs comprise at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, or each of the ratios 106a/140-5p, 106a/142-3p, 126/140-5p, 126/142-3p, 133a/142-3p, 140-5p/17, 142-3p/148a, 142-3p/15b, 142-3p/17, 142-3p/21, 142-3p/221, 142-3p/30b, 320/660, 106a/660, 106a/92a, 126/660, 140-5p/197, 140-5p/28-3p, 142-3p/145, 142-3p/197, 142-3p/28-3p, 17/660, 17/92a, 197/660, 197/92a, 19b/660, and 28-3p/660, or the inverse ratios thereof. The skilled artisan would understand that utilizing some but not all of these 27 ratios would be expected to provide an assay that accurately identifies the presence of a tumor.

In some embodiments, the invention provides the use of a plurality of primers or probes useful for detecting any of 27 miRNA pairs in the manufacture of a diagnostic reagent useful in determining the presence of a pulmonary tumor in a subject, wherein the detection comprises:
(a) determine the expression ratio of an miRNA pair in a biological sample from the subject;
(b) compare the expression ratio from step (a) with a cut-off value determined from the average ratio of a plurality of corresponding miRNA pairs from a plurality of control samples;
(c) assign a positive score for the expression ratio of the miRNA pair in step (a) if the ratio exceeds the cut-off value in step (b), or assign a non-positive score for the expression ratio of the miRNA pair in step (a) if the ratio does not exceed the cut-off value in step (b);
(d) repeat steps (a) through (c) for additional miRNA pairs until either (1) at least nine miRNA pair expression ratios are assigned a positive score, or (2) after the comparison of the expression ratios of 27 miRNA pairs less than nine miRNA pair expression ratios are assigned a positive score,
   wherein the miRNA pairs comprise 106a/140-5p, 106a/142-3p, 126/140-5p, 126/142-3p, 133a/142-3p, 140-5p/17, 142-3p/148a, 142-3p/15b, 142-3p/17, 142-3p/21, 142-3p/221, 142-3p/30b, or 320/660, or the inverse ratios thereof; and
(e) categorize the presence of a pulmonary tumor as (i) positive if at least nine of the miRNA pair expression ratios are assigned a positive score, or (ii) negative if less than nine miRNA pair expression ratios are assigned a positive score.

In some embodiments, the plurality of primers or probes are useful for detecting more than 2, more than 3, more than 4, more than 5, more than 6, more than 7, more than 8, more than 9, more than 10, more than 11, more than 12, or each of the miRNA pair expression ratios 106a/140-5p, 106a/142-3p, 126/140-5p, 126/142-3p, 133a/142-3p, 140-5p/17, 142-3p/148a, 142-3p/15b, 142-3p/17, 142-3p/21, 142-3p/221, 142-3p/30b, and 320/660, or the inverse ratios thereof.

In other embodiments, the plurality of primers or probes are further useful for detecting the miRNA pair expression ratios 106a/660, 106a/92a, 126/660, 140-5p/197, 140-5p/28-3p, 142-3p/145, 142-3p/197, 142-3p/28-3p, 17/660, 17/92a, 197/660, 197/92a, 19b/660, or 28-3p/660, or the inverse ratios thereof.

In still other embodiments, the plurality of primers or probes are useful for detecting at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, or each of the miRNA pair expression ratios 106a/140-5p, 106a/142-3p, 126/140-5p, 126/142-3p, 133a/142-3p, 140-5p/17, 142-3p/148a, 142-3p/15b, 142-3p/17, 142-3p/21, 142-3p/221, 142-3p/30b, 320/660, 106a/660, 106a/92a, 126/660, 140-5p/197, 140-5p/28-3p, 142-3p/145, 142-3p/197, 142-3p/28-3p, 17/660, 17/92a, 197/660, 197/92a, 19b/660, and 28-3p/660, or the inverse ratios thereof. The skilled artisan would understand that utilizing some but not all of these 27 ratios would be expected to provide an assay that accurately identifies the presence of a tumor.

In some embodiments, at least one primer or probe in the plurality of primers and probes is capable of selectively binding to at least one miRNA of a miRNA pair in a sample. In some embodiments, the plurality of primers or probes comprises at least one primer or probe capable of selectively binding to at least one miRNA of at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, or each of the miRNA pair expression ratios 106a/140-5p, 106a/142-3p, 126/140-5p, 126/142-3p, 133a/142-3p, 140-5p/17, 142-3p/148a, 142-3p/15b, 142-3p/17, 142-3p/21, 142-3p/221, 142-3p/30b, 320/660, 106a/660, 106a/92a, 126/660, 140-5p/197, 140-5p/28-3p, 142-3p/145, 142-3p/197, 142-3p/28-3p, 17/660, 17/92a, 197/660, 197/92a, 19b/660, and 28-3p/660 in a sample.

In some embodiments, the invention provides a kit for determining the presence of a pulmonary tumor in a subject. The kit comprises a plurality of primers or probes useful for detecting any of 27 miRNA pairs in the manufacture of a diagnostic reagent and instructions for determining the presence of a pulmonary tumor in a subject utilizing the plurality of primers or probes. The instructions comprise:
(a) determine the expression ratio of an miRNA pair in a biological sample from the subject;
(b) compare the expression ratio from step (a) with a cut-off value determined from the average ratio of a plurality of corresponding miRNA pairs from a plurality of control samples;
(c) assign a positive score for the expression ratio of the miRNA pair in step (a) if the ratio exceeds the cut-off value in step (b), or assign a non-positive score for the expression ratio of the miRNA pair in step (a) if the ratio does not exceed the cut-off value in step (b);
(d) repeat steps (a) through (c) for additional miRNA pairs until either (1) at least nine miRNA pair expression ratios are assigned a positive score, or (2) after the comparison of the expression ratios of 27 miRNA pairs less than nine miRNA pair expression ratios are assigned a positive score,
   wherein the miRNA pairs comprise 106a/140-5p, 106a/142-3p, 126/140-5p, 126/142-3p, 133a/142-3p, 140-5p/17, 142-3p/148a, 142-3p/15b, 142-3p/17, 142-3p/21, 142-3p/221, 142-3p/30b, or 320/660, or the inverse ratios thereof; and
(e) categorize the presence of a pulmonary tumor as (i) positive if at least nine of the miRNA pair expression ratios are assigned a positive score, or (ii) negative if less than nine miRNA pair expression ratios are assigned a positive score.

In some embodiments, the plurality of primers or probes provided in the kit are useful for detecting more than 2, more than 3, more than 4, more than 5, more than 6, more than 7, more than 8, more than 9, more than 10, more than 11, more than 12, or each of the miRNA pair expression ratios 106a/140-5p, 106a/142-3p, 126/140-5p, 126/142-3p, 133a/142-3p, 140-5p/17, 142-3p/148a, 142-3p/15b, 142-3p/17, 142-3p/21, 142-3p/221, 142-3p/30b, and 320/660, or the inverse ratios thereof.

In other embodiments, the plurality of primers or probes provided in the kit are further useful for detecting the miRNA pair expression ratios 106a/660, 106a/92a, 126/660, 140-5p/197, 140-5p/28-3p, 142-3p/145, 142-3p/197, 142-3p/28-3p, 17/660, 17/92a, 197/660, 197/92a, 19b/660, or 28-3p/660, or the inverse ratios thereof.

In still other embodiments, the plurality of primers or probes provided in the kit are useful for detecting at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, or each of the miRNA pair expression ratios 106a/140-5p, 106a/142-3p, 126/140-5p, 126/142-3p, 133a/142-3p, 140-5p/17, 142-3p/148a, 142-3p/15b, 142-3p/17, 142-3p/21, 142-3p/221, 142-3p/30b, 320/660, 106a/660, 106a/92a, 126/660, 140-5p/197, 140-5p/28-3p, 142-3p/145, 142-3p/197, 142-3p/28-3p, 17/660, 17/92a, 197/660, 197/92a, 19b/660, and 28-3p/660, or the inverse ratios thereof.

In some embodiments, at least one primer or probe in the plurality of primers and probes provided in the kit is capable of selectively binding to at least one miRNA of a miRNA pair in a sample. In some embodiments, the plurality of primers or probes provided in the kit comprises at least one primer or probe capable of selectively binding to at least one miRNA of at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, or each of the miRNA pair expression ratios 106a/140-5p, 106a/142-3p, 126/140-5p, 126/142-3p, 133a/142-3p, 140-5p/17, 142-3p/148a, 142-3p/15b, 142-3p/17, 142-3p/21, 142-3p/221, 142-3p/30b, 320/660, 106a/660, 106a/92a, 126/660, 140-5p/197, 140-5p/28-3p, 142-3p/145, 142-3p/197, 142-3p/28-3p, 17/660, 17/92a, 197/660, 197/92a, 19b/660, and 28-3p/660 in a sample.

### "Presence of Aggressive Pulmonary Tumor" Signature

In other embodiments, a method of determining the presence of an aggressive pulmonary tumor in a subject is provided. The method comprises:
(a) determine the expression ratio of an miRNA pair in a biological sample from the subject;
(b) compare the expression ratio from step (a) with a cut-off value determined from the average ratio of a plurality of corresponding miRNA pairs from a plurality of control samples;
(c) assign a positive score for the expression ratio of the miRNA pair in step (a) if the ratio exceeds the cut-off value in step (b), or assign a non-positive score for the expression ratio of the miRNA pair in step (a) if the ratio does not exceed the cut-off value in step (b);
(d) repeat steps (a) through (c) for additional miRNA pairs until either (1) at least fourteen miRNA pair expression ratios are assigned a positive score, or (2) after the comparison of the expression ratios of 28 miRNA pairs less than fourteen miRNA pair expression ratios are assigned a positive score,
   wherein the miRNA pairs comprise 106a/16, 106/660, 16/17, 16/320, 17/660, 197/30b, 197/30c, 320/451, 320/486-5p, or 320/660, or the inverse ratios thereof; and
(e) categorize the presence of an aggressive pulmonary tumor as (i) positive if at least fourteen of the miRNA pair expression ratios are assigned a positive score, or (ii) negative if less than fourteen miRNA pair expression ratios are assigned a positive score.

In some embodiments, the miRNA pairs of this assay comprise more than 2, more than 3, more than 4, more than 5, more than 6, more than 7, more than 8, more than 9, or each of the ratios 106a/16, 106/660, 16/17, 16/320, 17/660, 197/30b, 197/30c, 320/451, 320/486-5p, and 320/660, or the inverse ratios thereof.

In other embodiments, the miRNA pairs can further comprise the ratios 106a/451, 106a/486-5p, 126/451, 126/486-5p, 126/660, 140-5p/197, 16/197, 17/451, 17/486-5p, 197/451, 197/486-5p, 197/660, 197/92a, 19b/451, 19b/486-5p, 19b/660, 28-3p/451, or 28-3p/486-5p, or the inverse ratios thereof.

In still other embodiments, the miRNA pairs comprise at least 14, at least 15, at least 16, at least 17, at least 18, at least 19, at least 20, at least 21, at least 22, at least 23, at least 24, at least 25, at least 26, at least 27, or each of the ratios 106a/16, 106/660, 16/17, 16/320, 17/660, 197/30b, 197/30c, 320/451, 320/486-5p, 320/660, 106a/451, 106a/486-5p, 126/451, 126/486-5p, 126/660, 140-5p/197, 16/197, 17/451, 17/486-5p, 197/451, 197/486-5p, 197/660, 197/92a, 19b/451, 19b/486-5p, 19b/660, 28-3p/451, or 28-3p/486-5p, or the inverse ratios thereof. The skilled artisan would understand that utilizing some but not all of these 28 ratios would be expected to provide an assay that accurately identifies the presence of an aggressive tumor.

In other embodiments, the invention provides the use of a plurality of primers or probes useful for detecting any of 28 miRNA pairs in the manufacture of a diagnostic reagent useful in determining the presence of an aggressive pulmonary tumor in a subject, wherein the detection comprises:
(a) determine the expression ratio of an miRNA pair in a biological sample from the subject;
(b) compare the expression ratio from step (a) with a cut-off value determined from the average ratio of a plurality of corresponding miRNA pairs from a plurality of control samples;
(c) assign a positive score for the expression ratio of the miRNA pair in step (a) if the ratio exceeds the cut-off value in step (b), or assign a non-positive score for the expression ratio of the miRNA pair in step (a) if the ratio does not exceed the cut-off value in step (b);
(d) repeat steps (a) through (c) for additional miRNA pairs until either (1) at least fourteen miRNA pair expression ratios are assigned a positive score, or (2) after the comparison of the expression ratios of 28 miRNA pairs less than fourteen miRNA pair expression ratios are assigned a positive score,
   wherein the miRNA pairs comprise 106a/16, 106/660, 16/17, 16/320, 17/660, 197/30b, 197/30c, 320/451, 320/486-5p, or 320/660, or the inverse ratios thereof; and
(e) categorize the presence of an aggressive pulmonary tumor as (i) positive if at least fourteen of the miRNA pair expression ratios are assigned a positive score, or (ii) negative if less than fourteen miRNA pair expression ratios are assigned a positive score.

In some embodiments, the plurality of primers or probes are useful for detecting more than 2, more than 3, more than 4, more than 5, more than 6, more than 7, more than 8, more than 9, or each of the miRNA pair expression ratios 106a/16, 106/660, 16/17, 16/320, 17/660, 197/30b, 197/30c, 320/451, 320/486-5p, and 320/660, or the inverse ratios thereof.

In other embodiments, the plurality of primers or probes are further useful for detecting the miRNA pair expression ratios 106a/451, 106a/486-5p, 126/451, 126/486-5p, 126/660, 140-5p/197, 16/197, 17/451, 17/486-5p, 197/451, 197/486-5p, 197/660, 197/92a, 19b/451, 19b/486-5p, 19b/660, 28-3p/451, or 28-3p/486-5p, or the inverse ratios thereof.

In still other embodiments, the plurality of primers or probes are useful for detecting at least 14, at least 15, at least 16, at least 17, at least 18, at least 19, at least 20, at least 21, at least 22, at least 23, at least 24, at least 25, at least 26, at least 27, or each of the miRNA pair expression ratios 106a/16, 106/660, 16/17, 16/320, 17/660, 197/30b, 197/30c, 320/451, 320/486-5p, 320/660, 106a/451, 106a/486-5p, 126/451, 126/486-5p, 126/660, 140-5p/197, 16/197, 17/451, 17/486-5p, 197/451, 197/486-5p, 197/660, 197/92a, 19b/451, 19b/486-5p, 19b/660, 28-3p/451, or 28-3p/486-5p, or the inverse ratios thereof. The skilled artisan would understand that utilizing some but not all of these 28 ratios would be expected to provide an assay that accurately identifies the presence of an aggressive tumor.

In some embodiments, at least one primer or probe in the plurality of primers and probes is capable of selectively binding to at least one miRNA of a miRNA pair in a sample. In some embodiments, the plurality of primers or probes comprises at least one primer or probe capable of selectively binding to at least one miRNA of at least 14, at least 15, at least 16, at least 17, at least 18, at least 19, at least 20, at least 21, at least 22, at least 23, at least 24, at least 25, at least 26, at least 27, or each of the miRNA pair expression ratios 106a/16, 106/660, 16/17, 16/320, 17/660, 197/30b, 197/30c, 320/451, 320/486-5p, 320/660, 106a/451, 106a/486-5p, 126/451, 126/486-5p, 126/660, 140-5p/197, 16/197, 17/451, 17/486-5p, 197/451, 197/486-5p, 197/660, 197/92a, 19b/451, 19b/486-5p, 19b/660, 28-3p/451, or 28-3p/486-5p in a sample.

In other embodiments, the invention provides a kit for determining the presence of an aggressive pulmonary tumor in a subject. The kit comprises a plurality of primers or probes useful for detecting any of 28 miRNA pairs in the manufacture of a diagnostic reagent and instructions for determining the presence of an aggressive pulmonary tumor in a subject utilizing the plurality of primers or probes. The instructions comprise:
(a) determine the expression ratio of an miRNA pair in a biological sample from the subject;
(b) compare the expression ratio from step (a) with a cut-off value determined from the average ratio of a plurality of corresponding miRNA pairs from a plurality of control samples;
(c) assign a positive score for the expression ratio of the miRNA pair in step (a) if the ratio exceeds the cut-off value in step (b), or assign a non-positive score for the expression ratio of the miRNA pair in step (a) if the ratio does not exceed the cut-off value in step (b);
(d) repeat steps (a) through (c) for additional miRNA pairs until either (1) at least fourteen miRNA pair expression ratios are assigned a positive score, or (2) after the comparison of the expression ratios of 28 miRNA pairs less than fourteen miRNA pair expression ratios are assigned a positive score,
   wherein the miRNA pairs comprise 106a/16, 106/660, 16/17, 16/320, 17/660, 197/30b, 197/30c, 320/451, 320/486-5p, or 320/660, or the inverse ratios thereof; and
(e) categorize the presence of an aggressive pulmonary tumor as (i) positive if at least fourteen of the miRNA pair expression ratios are assigned a positive score, or (ii) negative if less than fourteen miRNA pair expression ratios are assigned a positive score.

In some embodiments, the plurality of primers or probes are useful for detecting more than 2, more than 3, more than 4, more than 5, more than 6, more than 7, more than 8, more than 9, or each of the miRNA pair expression ratios 106a/16, 106/660, 16/17, 16/320, 17/660, 197/30b, 197/30c, 320/451, 320/486-5p, and 320/660, or the inverse ratios thereof.

In other embodiments, the plurality of primers or probes are further useful for detecting the miRNA pair expression ratios 106a/451, 106a/486-5p, 126/451, 126/486-5p, 126/660, 140-5p/197, 16/197, 17/451, 17/486-5p, 197/451, 197/486-5p, 197/660, 197/92a, 19b/451, 19b/486-5p, 19b/660, 28-3p/451, or 28-3p/486-5p, or the inverse ratios thereof.

In still other embodiments, the plurality of primers or probes are useful for detecting at least 14, at least 15, at least 16, at least 17, at least 18, at least 19, at least 20, at least 21, at least 22, at least 23, at least 24, at least 25, at least 26, at least 27, or each of the miRNA pair expression ratios 106a/16, 106/660, 16/17, 16/320, 17/660, 197/30b, 197/30c, 320/451, 320/486-5p, 320/660, 106a/451, 106a/486-5p, 126/451, 126/486-5p, 126/660, 140-5p/197, 16/197, 17/451, 17/486-5p, 197/451, 197/486-5p, 197/660, 197/92a, 19b/451, 19b/486-5p, 19b/660, 28-3p/451, or 28-3p/486-5p, or the inverse ratios thereof.

In some embodiments, at least one primer or probe in the plurality of primers and probes provided in the kit is capable of selectively binding to at least one miRNA of a miRNA pair in a sample. In some embodiments, the plurality of primers or probes provided in the kit comprises at least one primer or probe capable of selectively binding to at least one miRNA of at least 14, at least 15, at least 16, at least 17, at least 18, at least 19, at least 20, at least 21, at least 22, at least 23, at least 24, at least 25, at least 26, at least 27, or each of the miRNA pair expression ratios 106a/16, 106/660, 16/17, 16/320, 17/660, 197/30b, 197/30c, 320/451, 320/486-5p, 320/660, 106a/451, 106a/486-5p, 126/451, 126/486-5p, 126/660, 140-5p/197, 16/197, 17/451, 17/486-5p, 197/451, 197/486-5p, 197/660, 197/92a, 19b/451, 19b/486-5p, 19b/660, 28-3p/451, or 28-3p/486-5p in a sample.

Along with the two diagnostic signatures ("presence of pulmonary tumor" and "presence of aggressive pulmonary tumor") described above, two prognostic signatures are provided - a "risk of manifesting a pulmonary tumor" signature, and a "risk of manifesting an aggressive pulmonary tumor" signature. These prognostic signatures can be utilized for determining risk within any time period after the sample is taken, e.g., 3 months, 6 months, 12 months, 18 months, 24 months, 36 months, or any time outside or in-between those time periods.

### "Risk of Manifesting a Pulmonary Tumor" Signature

Thus, in further embodiments, a method of determining the risk of manifesting a pulmonary tumor in a subject is provided. The method comprises:
(a) determine the expression ratio of an miRNA pair in a biological sample from the subject;
(b) compare the expression ratio from step (a) with a cut-off value determined from the average ratio of a plurality of corresponding miRNA pairs from a plurality of control samples;
(c) assign a positive score for the expression ratio of the miRNA pair in step (a) if the ratio exceeds the cut-off value in step (b), or assign a non-positive score for the expression ratio of the miRNA pair in step (a) if the ratio does not exceed the cut-off value in step (b);
(d) repeat steps (a) through (c) for additional miRNA pairs until either (1) at least ten miRNA pair expression ratios are assigned a positive score, or (2) after the comparison of the expression ratios of 27 miRNA pairs, less than ten miRNA pair expression ratios are assigned a positive score,
   wherein the miRNA pairs comprise 133a/92a, 15b/21, 15b/30b, 15b/30c, 16/197, or 28-3p/451, or the inverse ratios thereof; and
(e) categorize the risk of manifesting a pulmonary tumor as (i) positive if at least ten miRNA pair expression ratios are assigned a positive score, or (ii) negative if less than ten miRNA expression ratios are assigned a positive score.

In some embodiments, the miRNA pairs of this assay comprise more than 2, more than 3, more than 4, more than 5, or each of the ratios 133a/92a, 15b/21, 15b/30b, 15b/30c, 16/197, and 28-3p/451, or the inverse ratios thereof.

In other embodiments, the miRNA pairs can further comprise the ratios 101/140-3p, 106a/451, 106a/660, 106a/92a, 126/660, 133a/451, 133a/660, 140-3p/660, 142-3p/15b, 15b/451, 15b/660, 17/451, 17/660, 17/92a, 197/19b, 197/451, 197/660, 197/92a, 19b/660, 28-3p/660, or 320/660, or the inverse ratios thereof.

In still other embodiments, the miRNA pairs comprise at least 10, at least 11, at least 12, at least 13, at least 14, at least 15, at least 16, at least 17, at least 18, at least 19, at least 20, at least 21, at least 22, at least 23, at least 24, at least 25, at least 26, or each of the ratios 133a/92a, 15b/21, 15b/30b, 15b/30c, 16/197, 28-3p/451, 101/140-3p, 106a/451, 106a/660, 106a/92a, 126/660, 133a/451, 133a/660, 140-3p/660, 142-3p/15b, 15b/451, 15b/660, 17/451, 17/660, 17/92a, 197/19b, 197/451, 197/660, 197/92a, 19b/660, 28-3p/660, or 320/660, or the inverse ratios thereof. The skilled artisan would understand that utilizing some but not all of these 27 ratios would be expected to provide an assay that accurately identifies the risk of manifesting a pulmonary tumor.

In some embodiments, the invention provides the use of a plurality of primers or probes useful for detecting any of 27 miRNA pairs in the manufacture of a diagnostic or prognostic reagent useful in determining the risk of manifesting a pulmonary tumor in a subject, wherein the detection comprises:
(a) determine the expression ratio of an miRNA pair in a biological sample from the subject;
(b) compare the expression ratio from step (a) with a cut-off value determined from the average ratio of a plurality of corresponding miRNA pairs from a plurality of control samples;
(c) assign a positive score for the expression ratio of the miRNA pair in step (a) if the ratio exceeds the cut-off value in step (b), or assign a non-positive score for the expression ratio of the miRNA pair in step (a) if the ratio does not exceed the cut-off value in step (b);
(d) repeat steps (a) through (c) for additional miRNA pairs until either (1) at least ten miRNA pair expression ratios are assigned a positive score, or (2) after the comparison of the expression ratios of 27 miRNA pairs, less than ten miRNA pair expression ratios are assigned a positive score,
   wherein the miRNA pairs comprise 133a/92a, 15b/21, 15b/30b, 15b/30c, 16/197, or 28-3p/451, or the inverse ratios thereof; and
(e) categorize the risk of manifesting a pulmonary tumor as (i) positive if at least ten miRNA pair expression ratios are assigned a positive score, or (ii) negative if less than ten miRNA expression ratios are assigned a positive score.

In some embodiments, the plurality of primers or probes are useful for detecting more than 2, more than 3, more than 4, more than 5, or each of the miRNA pair expression ratios 133a/92a, 15b/21, 15b/30b, 15b/30c, 16/197, and 28-3p/451, or the inverse ratios thereof.

In other embodiments, the plurality of primers or probes are further useful for detecting the miRNA pair expression ratios 101/140-3p, 106a/451, 106a/660, 106a/92a, 126/660, 133a/451, 133a/660, 140-3p/660, 142-3p/15b, 15b/451, 15b/660, 17/451, 17/660, 17/92a, 197/19b, 197/451, 197/660, 197/92a, 19b/660, 28-3p/660, or 320/660, or the inverse ratios thereof.

In still other embodiments, the plurality of primers or probes are useful for detecting at least 10, at least 11, at least 12, at least 13, at least 14, at least 15, at least 16, at least 17, at least 18, at least 19, at least 20, at least 21, at least 22, at least 23, at least 24, at least 25, at least 26, or each of the miRNA pair expression ratios 133a/92a, 15b/21, 15b/30b, 15b/30c, 16/197, 28-3p/451, 101/140-3p, 106a/451, 106a/660, 106a/92a, 126/660, 133a/451, 133a/660, 140-3p/660, 142-3p/15b, 15b/451, 15b/660, 17/451, 17/660, 17/92a, 197/19b, 197/451, 197/660, 197/92a, 19b/660, 28-3p/660, or 320/660, or the inverse ratios thereof. The skilled artisan would understand that utilizing some but not all of these 27 ratios would be expected to provide an assay that accurately identifies the risk of manifesting a pulmonary tumor.

In some embodiments, at least one primer or probe in the plurality of primers and probes is capable of selectively binding to at least one miRNA of a miRNA pair in a sample. In some embodiments, the plurality of primers or probes comprises at least one primer or probe capable of selectively binding to at least one miRNA of at least 10, at least 11, at least 12, at least 13, at least 14, at least 15, at least 16, at least 17, at least 18, at least 19, at least 20, at least 21, at least 22, at least 23, at least 24, at least 25, at least 26, or each of the miRNA pair expression ratios 133a/92a, 15b/21, 15b/30b, 15b/30c, 16/197, 28-3p/451, 101/140-3p, 106a/451, 106a/660, 106a/92a, 126/660, 133a/451, 133a/660, 140-3p/660, 142-3p/15b, 15b/451, 15b/660, 17/451, 17/660, 17/92a, 197/19b, 197/451, 197/660, 197/92a, 19b/660, 28-3p/660, or 320/660 in a sample.

In other embodiments, the invention provides a kit for determining the risk of manifesting a pulmonary tumor in a subject. The kit comprises a plurality of primers or probes useful for detecting any of 27 miRNA pairs in the manufacture of a diagnostic or prognostic reagent and instructions for determining the risk of manifesting a pulmonary tumor in a subject utilizing the plurality of primers or probes. The instructions comprise:
(a) determine the expression ratio of an miRNA pair in a biological sample from the subject;
(b) compare the expression ratio from step (a) with a cut-off value determined from the average ratio of a plurality of corresponding miRNA pairs from a plurality of control samples;
(c) assign a positive score for the expression ratio of the miRNA pair in step (a) if the ratio exceeds the cut-off value in step (b), or assign a non-positive score for the expression ratio of the miRNA pair in step (a) if the ratio does not exceed the cut-off value in step (b);
(d) repeat steps (a) through (c) for additional miRNA pairs until either (1) at least ten miRNA pair expression ratios are assigned a positive score, or (2) after the comparison of the expression ratios of 27 miRNA pairs, less than ten miRNA pair expression ratios are assigned a positive score,
   wherein the miRNA pairs comprise 133a/92a, 15b/21, 15b/30b, 15b/30c, 16/197, or 28-3p/451, or the inverse ratios thereof; and
(e) categorize the risk of manifesting a pulmonary tumor as (i) positive if at least ten miRNA pair expression ratios are assigned a positive score, or (ii) negative if less than ten miRNA expression ratios are assigned a positive score.

In some embodiments, the plurality of primers or probes are useful for detecting more than 2, more than 3, more than 4, more than 5, or each of the miRNA pair expression ratios 133a/92a, 15b/21, 15b/30b, 15b/30c, 16/197, and 28-3p/451, or the inverse ratios thereof.

In other embodiments, the plurality of primers or probes are further useful for detecting the miRNA pair expression ratios 101/140-3p, 106a/451, 106a/660, 106a/92a, 126/660, 133a/451, 133a/660, 140-3p/660, 142-3p/15b, 15b/451, 15b/660, 17/451, 17/660, 17/92a, 197/19b, 197/451, 197/660, 197/92a, 19b/660, 28-3p/660, or 320/660, or the inverse ratios thereof.

In still other embodiments, the plurality of primers or probes are useful for detecting at least 10, at least 11, at least 12, at least 13, at least 14, at least 15, at least 16, at least 17, at least 18, at least 19, at least 20, at least 21, at least 22, at least 23, at least 24, at least 25, at least 26, or each of the miRNA pair expression ratios 133a/92a, 15b/21, 15b/30b, 15b/30c, 16/197, 28-3p/451, 101/140-3p, 106a/451, 106a/660, 106a/92a, 126/660, 133a/451, 133a/660, 140-3p/660, 142-3p/15b, 15b/451, 15b/660, 17/451, 17/660, 17/92a, 197/19b, 197/451, 197/660, 197/92a, 19b/660, 28-3p/660, or 320/660, or the inverse ratios thereof.

In some embodiments, at least one primer or probe in the plurality of primers and probes provided in the kit is capable of selectively binding to at least one miRNA of a miRNA pair in a sample. In some embodiments, the plurality of primers or probes provided in the kit comprises at least one primer or probe capable of selectively binding to at least one miRNA of at least 10, at least 11, at least 12, at least 13, at least 14, at least 15, at least 16, at least 17, at least 18, at least 19, at least 20, at least 21, at least 22, at least 23, at least 24, at least 25, at least 26, or each of the miRNA pair expression ratios 133a/92a, 15b/21, 15b/30b, 15b/30c, 16/197, 28-3p/451, 101/140-3p, 106a/451, 106a/660, 106a/92a, 126/660, 133a/451, 133a/660, 140-3p/660, 142-3p/15b, 15b/451, 15b/660, 17/451, 17/660, 17/92a, 197/19b, 197/451, 197/660, 197/92a, 19b/660, 28-3p/660, or 320/660 in a sample.

### "Risk of Manifesting an Aggressive Pulmonary Tumor" Signature

A method of determining the risk of manifesting an aggressive pulmonary tumor in a subject is also provided herewith. The method comprises:
(a) determine the expression ratio of an miRNA pair in a biological sample from the subject;
(b) compare the expression ratio from step (a) with a cut-off value determined from the average ratio of a plurality of corresponding miRNA pairs from a plurality of control samples;
(c) assign a positive score for the expression ratio of the miRNA pair in step (a) if the ratio exceeds the cut-off value in step (b), or assign a non-positive score for the expression ratio of the miRNA pair in step (a) if the ratio does not exceed the cut-off value in step (b);
(d) repeat steps (a) through (c) for additional miRNA pairs until either (1) at least fourteen miRNA pair expression ratios are assigned a positive score, or (2) after the comparison of the expression ratios of 28 miRNA pairs less than fourteen miRNA pair expression ratios are assigned a positive score,
   wherein the miRNA pairs comprise 106a/142-3p, 126/142-3p, 126/21, 126/92a, 142-3p/17, 142-3p/197, 142-3p/28-3p, 197/19b, 197/660, or 28-3p/660, or the inverse ratios thereof; and
(e) categorize the risk of manifesting an aggressive pulmonary tumor as (i) positive if at least fourteen miRNA pair expression ratios are assigned a positive score, or (ii) negative if less than fourteen miRNA pair expression ratios are assigned a positive score.

In some embodiments, the miRNA pairs of this assay comprise more than 2, more than 3, more than 4, more than 5, more than 6, more than 7, more than 8, more than 9, or each of the ratios 106a/142-3p, 126/142-3p, 126/21, 126/92a, 142-3p/17, 142-3p/197, 142-3p/28-3p, 197/19b, 197/660, and 28-3p/660, or the inverse ratios thereof.

In other embodiments, the miRNA pairs can further comprise the ratios 106a/451, 126/451, 145/197, 17/451, 197/21, 197/30b, 197/30c, 197/451, 197/92a, 19b/451, 21/221, 21/28-3p, 28-3p/30b, 28-3p/30c, 28-3p/451, 28-3p/92a, 320/451, or 320/92a, or the inverse ratios thereof.

In still other embodiments, the miRNA pairs comprise at least 14, at least 15, at least 16, at least 17, at least 18, at least 19, at least 20, at least 21, at least 22, at least 23, at least 24, at least 25, at least 26, at least 27, or each of the ratios 106a/142-3p, 126/142-3p, 126/21, 126/92a, 142-3p/17, 142-3p/197, 142-3p/28-3p, 197/19b, 197/660, 28-3p/660, 106a/451, 126/451, 145/197, 17/451, 197/21, 197/30b, 197/30c, 197/451, 197/92a, 19b/451, 21/221, 21/28-3p, 28-3p/30b, 28-3p/30c, 28-3p/451, 28-3p/92a, 320/451, and 320/92a, or the inverse ratios thereof. The skilled artisan would understand that utilizing some but not all of these 28 ratios would be expected to provide an assay that accurately identifies the risk of manifesting an aggressive tumor.

In some embodiments, the invention provides the use of a plurality of primers or probes useful for detecting any of 28 miRNA pairs in the manufacture of a diagnostic or prognostic reagent useful in determining the risk of manifesting an aggressive pulmonary tumor in a subject, wherein the detection comprises:
(a) determine the expression ratio of an miRNA pair in a biological sample from the subject;
(b) compare the expression ratio from step (a) with a cut-off value determined from the average ratio of a plurality of corresponding miRNA pairs from a plurality of control samples;
(c) assign a positive score for the expression ratio of the miRNA pair in step (a) if the ratio exceeds the cut-off value in step (b), or assign a non-positive score for the expression ratio of the miRNA pair in step (a) if the ratio does not exceed the cut-off value in step (b);
(d) repeat steps (a) through (c) for additional miRNA pairs until either (1) at least fourteen miRNA pair expression ratios are assigned a positive score, or (2) after the comparison of the expression ratios of 28 miRNA pairs less than fourteen miRNA pair expression ratios are assigned a positive score,
   wherein the miRNA pairs comprise 106a/142-3p, 126/142-3p, 126/21, 126/92a, 142-3p/17, 142-3p/197, 142-3p/28-3p, 197/19b, 197/660, or 28-3p/660, or the inverse ratios thereof; and
(e) categorize the risk of manifesting an aggressive pulmonary tumor as (i) positive if at least fourteen miRNA pair expression ratios are assigned a positive score, or (ii) negative if less than fourteen miRNA pair expression ratios are assigned a positive score.

In some embodiments, the plurality of primers or probes are useful for detecting more than 2, more than 3, more than 4, more than 5, more than 6, more than 7, more than 8, more than 9, or each of the miRNA pair expression ratios 106a/142-3p, 126/142-3p, 126/21, 126/92a, 142-3p/17, 142-3p/197, 142-3p/28-3p, 197/19b, 197/660, and 28-3p/660, or the inverse ratios thereof.

In other embodiments, the plurality of primers or probes are further useful for detecting the miRNA pair expression ratios 106a/451, 126/451, 145/197, 17/451, 197/21, 197/30b, 197/30c, 197/451, 197/92a, 19b/451, 21/221, 21/28-3p, 28-3p/30b, 28-3p/30c, 28-3p/451, 28-3p/92a, 320/451, or 320/92a, or the inverse ratios thereof.

In still other embodiments, the plurality of primers or probes are useful for detecting at least 14, at least 15, at least 16, at least 17, at least 18, at least 19, at least 20, at least 21, at least 22, at least 23, at least 24, at least 25, at least 26, at least 27, or each of the miRNA pair expression ratios 106a/142-3p, 126/142-3p, 126/21, 126/92a, 142-3p/17, 142-3p/197, 142-3p/28-3p, 197/19b, 197/660, 28-3p/660, 106a/451, 126/451, 145/197, 17/451, 197/21, 197/30b, 197/30c, 197/451, 197/92a, 19b/451, 21/221, 21/28-3p, 28-3p/30b, 28-3p/30c, 28-3p/451, 28-3p/92a, 320/451, and 320/92a, or the inverse ratios thereof. The skilled artisan would understand that utilizing some but not all of these 28 ratios would be expected to provide an assay that accurately identifies the risk of manifesting an aggressive pulmonary tumor.

In other embodiments, the invention provides a kit for determining the risk of manifesting an aggressive pulmonary tumor in a subject. The kit comprises a plurality of primers or probes useful for detecting any of 28 miRNA pairs in the manufacture of a diagnostic or prognostic reagent and instructions for determining the risk of manifesting an aggressive pulmonary tumor in a subject utilizing the plurality of primers or probes. The instructions comprise:
(a) determine the expression ratio of an miRNA pair in a biological sample from the subject;
(b) compare the expression ratio from step (a) with a cut-off value determined from the average ratio of a plurality of corresponding miRNA pairs from a plurality of control samples;
(c) assign a positive score for the expression ratio of the miRNA pair in step (a) if the ratio exceeds the cut-off value in step (b), or assign a non-positive score for the expression ratio of the miRNA pair in step (a) if the ratio does not exceed the cut-off value in step (b);
(d) repeat steps (a) through (c) for additional miRNA pairs until either (1) at least fourteen miRNA pair expression ratios are assigned a positive score, or (2) after the comparison of the expression ratios of 28 miRNA pairs less than fourteen miRNA pair expression ratios are assigned a positive score,
   wherein the miRNA pairs comprise 106a/142-3p, 126/142-3p, 126/21, 126/92a, 142-3p/17, 142-3p/197, 142-3p/28-3p, 197/19b, 197/660, or 28-3p/660, or the inverse ratios thereof; and
(e) categorize the risk of manifesting an aggressive pulmonary tumor as (i) positive if at least fourteen miRNA pair expression ratios are assigned a positive score, or (ii) negative if less than fourteen miRNA pair expression ratios are assigned a positive score.

In some embodiments, the plurality of primers or probes are useful for detecting more than 2, more than 3, more than 4, more than 5, more than 6, more than 7, more than 8, more than 9, or each of the miRNA pair expression ratios 106a/142-3p, 126/142-3p, 126/21, 126/92a, 142-3p/17, 142-3p/197, 142-3p/28-3p, 197/19b, 197/660, and 28-3p/660, or the inverse ratios thereof.

In other embodiments, the plurality of primers or probes are further useful for detecting the miRNA pair expression ratios 106a/451, 126/451, 145/197, 17/451, 197/21, 197/30b, 197/30c, 197/451, 197/92a, 19b/451, 21/221, 21/28-3p, 28-3p/30b, 28-3p/30c, 28-3p/451, 28-3p/92a, 320/451, or 320/92a, or the inverse ratios thereof.

In still other embodiments, the plurality of primers or probes are useful for detecting at least 14, at least 15, at least 16, at least 17, at least 18, at least 19, at least 20, at least 21, at least 22, at least 23, at least 24, at least 25, at least 26, at least 27, or each of the miRNA pair expression ratios 106a/142-3p, 126/142-3p, 126/21, 126/92a, 142-3p/17, 142-3p/197, 142-3p/28-3p, 197/19b, 197/660, 28-3p/660, 106a/451, 126/451, 145/197, 17/451, 197/21, 197/30b, 197/30c, 197/451, 197/92a, 19b/451, 21/221, 21/28-3p, 28-3p/30b, 28-3p/30c, 28-3p/451, 28-3p/92a, 320/451, and 320/92a, or the inverse ratios thereof.

In some embodiments, at least one primer or probe in the plurality of primers and probes provided in the kit is capable of selectively binding to at least one miRNA of a miRNA pair in a sample. In some embodiments, the plurality of primers or probes provided in the kit comprises at least one primer or probe capable of selectively binding to at least one miRNA of at least 14, at least 15, at least 16, at least 17, at least 18, at least 19, at least 20, at least 21, at least 22, at least 23, at least 24, at least 25, at least 26, at least 27, or each of the miRNA pair expression ratios 106a/142-3p, 126/142-3p, 126/21, 126/92a, 142-3p/17, 142-3p/197, 142-3p/28-3p, 197/19b, 197/660, 28-3p/660, 106a/451, 126/451, 145/197, 17/451, 197/21, 197/30b, 197/30c, 197/451, 197/92a, 19b/451, 21/221, 21/28-3p, 28-3p/30b, 28-3p/30c, 28-3p/451, 28-3p/92a, 320/451, and 320/92a in a sample.

### "Risk of Developing or Having Pulmonary Tumor" Classifier

The above four methods can be combined in a method for predicting the risk of developing or having a pulmonary tumor in a subject. That method comprises:
(a) determining the presence of a pulmonary tumor signature described above;
(b) determining the presence of an aggressive pulmonary tumor signature described above;
(c) determining the risk of manifesting a pulmonary tumor signature described above;
(d) determining the risk of manifesting an aggressive pulmonary tumor signature described above;
(e) categorizing the subject as low risk of developing or having a pulmonary tumor if none of the signatures of steps (a)-(d) are categorized as positive;
(f) categorizing the subject as having an intermediate risk of developing or having a pulmonary tumor if at least the presence of a pulmonary tumor signature of step (a) is positive, or the risk of manifesting a pulmonary tumor signature of step (c) is positive; and both the presence of an aggressive pulmonary tumor signature of step (b) and the risk of manifesting an aggressive pulmonary tumor signature of step (d) are negative; and
(g) categorizing the subject as high risk of developing or having a pulmonary tumor if at least the presence of an aggressive pulmonary tumor signature of step (b) is positive or the risk of manifesting an aggressive pulmonary tumor signature of step (d) is positive.

The above four uses can be combined in a use for predicting the risk of developing or having a pulmonary tumor in a subject. That use comprises:
(a) using a plurality of primers or probes useful for detecting any of the 27 miRNA pairs in the manufacture of a diagnostic reagent useful in determining the presence of a pulmonary tumor signature described above;
(b) using a plurality of primers or probes useful for detecting any of the 28 miRNA pairs in the manufacture of a diagnostic reagent useful in determining the presence of an aggressive pulmonary tumor signature described above;
(c) using a plurality of primers or probes useful for detecting any of the 27 miRNA pairs in the manufacture of a diagnostic or prognostic reagent useful in determining the risk of manifesting a pulmonary tumor signature described above;
(d) using a plurality of primers or probes useful for detecting any of the 28 miRNA pairs in the manufacture of a diagnostic or prognostic reagent useful in determining the risk of manifesting an aggressive pulmonary tumor signature described above;
(e) categorizing the subject as low risk of developing or having a pulmonary tumor if none of the signatures of steps (a)-(d) are categorized as positive;
(f) categorizing the subject as having an intermediate risk of developing or having a pulmonary tumor if at least the presence of a pulmonary tumor signature of step (a) is positive, or the risk of manifesting a pulmonary tumor signature of step (c) is positive; and both the presence of an aggressive pulmonary tumor signature of step (b) and the risk of manifesting an aggressive pulmonary tumor signature of step (d) are negative; and
(g) categorizing the subject as high risk of developing or having a pulmonary tumor if at least the presence of an aggressive pulmonary tumor signature of step (b) is positive or the risk of manifesting an aggressive pulmonary tumor signature of step (d) is positive.

### A. Definitions

The term "pulmonary tumor" a used herein can be either a benign or malignant pulmonary tumor. The pulmonary tumor can be associated with one or more lung conditions and may take the form of, e.g., a pulmonary nodule or a pulmonary mass.

The term "lung condition" as used herein refers to a disease, event, or change in health status relating to the lung, including for example lung cancer and various non-cancerous conditions. Examples of non-cancerous lung condition include chronic obstructive pulmonary disease (COPD), benign pulmonary tumors or masses of cells (e.g., hamartoma, fibroma, neurofibroma), granuloma, sarcoidosis, and infections caused by bacterial (e.g., tuberculosis) or fungal (*e.g*., histoplasmosis) pathogens. In certain embodiments, a lung condition may be associated with the appearance of radiographic pulmonary nodules.

The above methods are not narrowly limited to diagnosing and/or prognosing any particular lung cancer. As used herein, "lung cancer" preferably refers to cancers of the lung, but may include any disease or other disorder of the respiratory system of a human or other mammal. Respiratory neoplastic disorders include, for example small cell carcinoma or small cell lung cancer (SCLC), non-small cell carcinoma or non-small cell lung cancer (NSCLC), squamous cell carcinoma (SCC), adenocarcinoma, broncho-alveolar carcinoma (BAC), mixed pulmonary carcinoma, malignant pleural mesothelioma, undifferentiated large cell carcinoma, giant cell carcinoma, synchronous tumors, large cell neuroendocrine carcinoma, adenosquamous carcinoma, undifferentiated carcinoma; and small cell carcinoma, including oat cell cancer, mixed small cell/large cell carcinoma (LC), large cell carcinoma, and combined small cell carcinoma; as well as adenoid cystic carcinoma, hamartomas, mucoepidermoid tumors, typical carcinoid lung tumors, atypical carcinoid lung tumors, peripheral carcinoid lung tumors, central carcinoid lung tumors, pleural mesotheliomas, undifferentiated pulmonary carcinoma and cancers that originate outside the lungs such as secondary cancers that have metastasized to the lungs from other parts of the body, or any other lung cancer now known or later discovered. Lung cancers may be of any stage or grade.

The term "biological sample from the subject" as used herein refers to a sample from any subject, including smokers, non-smokers, former smokers, cancer patients (e.g., for diagnosing and/or monitoring cancer recurrence after treatment), former cancer patients, etc. In specific embodiments, the biological sample originates from a smoker individual who, at the moment of the collection of the sample, does not present a pulmonary tumor if subjected to imaging diagnostic methods, in particular the smoker individual not presenting nodules of dimensions of greater than 5 mm if subjected to a spiral CT scan.

The term "biological sample" as used herein can be any tissue that provides an accurate measurement of the miRNA profile in the subject. In some embodiments, the biological sample is a biological fluid. These embodiments are not narrowly limited to any particular bodily fluid, since miRNA is present in essentially all bodily fluids (Weber et al., The microRNA spectrum in 12 body fluids, Clin Chem 56:1733-1741, 2010; De Guire et al., Clin Biochem 46:846-860, 2013; see also Rodriguez-Dorantes et al., Meth Mol Biol 1165:81-87, 2014). Examples of useful bodily fluids are peripheral blood, serum, plasma, ascites, urine, sputum, saliva, broncheoalveolar lavage fluid, cyst fluid, pleural fluid, peritoneal fluid, lymph, pus, lavage fluids from sinus cavities, bronchopulmonary aspirates, and bone marrow aspirates. The skilled artisan can determine, without undue experimentation, whether any particular bodily fluid is useful for any particular application. In some embodiments, the bodily fluid is plasma or serum. In some embodiments, the biological sample is a tissue sample.

As used herein, the singular forms "a", "an" and "the" are intended to include the plural forms as well, unless the context clearly indicates otherwise. Additionally, the use of "or" is intended to include "and/or", unless the context clearly indicates otherwise.

As used herein, an "individual", "subject", "patient" or "subject in need thereof" is an individual having an risk of developing a tumor or an aggressive tumor or one who may have or may be afflicted with, or diagnosed as having, a tumor or aggressive tumor. These terms may be utilized interchangeably. Preferably, the individual is a mammal. The mammal can be e.g., any mammal, e.g., a human, primate, bird, mouse, rat, fowl, dog, cat, cow, horse, goat, camel, sheep or a pig. Preferably, the mammal is a human.

As used herein, a "primer" or a "probe" in the plurality of primers or probes is a nucleic acid molecule. The primer or probe can be DNA, RNA, or cDNA. The primer or probe can be naturally occurring or synthetic. The primer or probe can be at least 5, at least 10, at least 15, at least 20, at least 25, at least 50, at least 75, at least 100 nucleic acids in length. Preferably, at least one primer or probe in the plurality of primers and probes is capable of selectively binding to at least one miRNA of a miRNA pair in a sample. Preferably, the plurality of primers or probes comprises at least one primer or probe capable of selectively binding to at least one miRNA of all the miRNA pair expression rations in a sample. The term "selectively binding" means that the primer or probe has a high affinity for binding to at least one miRNA of a miRNA pair in a sample or a higher affinity for binding to at least one miRNA of a miRNA pair as compared to any other miRNA in a sample. The term "primer" means that the nucleic acid molecule is capable of serving as a starting point for DNA synthesis. That is, DNA polymerases can initiate replication from the 3' end of a primer.

With any of the above methods, the term "the expression ratio of an miRNA pair" as used herein is the ratio of the expression level of two specific miRNAs. It should be appreciated that the ratios for any of the miRNA pairs specified in any of the methods described herein could be calculated as the inverse ratio of that specified. As a nonlimiting example, when the miRNA pairs for a particular method are "106a/140-5p, 106a/142-3p, 126/140-5p, 126/142-3p, 133a/142-3p, 140-5p/17, 142-3p/148a, 142-3p/15b, 142-3p/17, 142-3p/21, 142-3p/221, 142-3p/30b, and 320/660, or the inverse ratios thereof," the expression ratio of any one, two, three, four, five, six, seven, eight, nine, ten, eleven, twelve or thirteen of these miRNA pairs could be calculated as the inverse ratio, where the cut-off values are adjusted accordingly.

The term "cut-off value" as used herein can be a negative or a positive number. With some ratios, an expression ratio that "exceeds the cut-off value" is a value that is lesser in magnitude than the cut-off value. With other ratios, an expression ratio that "exceeds the cut-off value" is a value that is greater in magnitude than the cut-off value. See, e.g., Table 9, where ">" indicates that a number greater in magnitude than the cut-off value signifies a ratio that exceeds the cut-off, and "<" indicate that a number lesser in magnitude than the cut-off value signifies a ratio that exceeds the cut-off value.

Under this definition of "exceeds the cut-off value," (i) a value of -4.50 for the ratio 133a/92a exceeds the cut-off value of -4.18 listed in Table 9, (ii) a value of 3.50 for the ratio 17/451 exceeds the cutoff-off value of 3.33 listed in Table 9, (iii) a value of -1.80 for the ratio 142-3p/17 exceeds the cut-off value of -1.95 listed in Table 9, and (iv) a value of 4.50 for the ratio 16/197 exceeds the cut-off value of 5.00 listed in Table 9.

In any of the methods described herein, the cut-off value for the expression ratio of any particular miRNA pair is determined from the average ratio of a plurality of corresponding miRNA pairs from a plurality of control samples. The term "plurality of control samples" as used herein can be from any defined population, for example healthy subjects without a history of any cancer, healthy subjects without a history of lung cancer, subjects that have lung cancer, subjects that never smoked, subjects that have smoked but no longer smoke, etc. In a preferred embodiment, the plurality of control samples are from healthy subjects and the cut-off value is the average ratio of the plurality of corresponding miRNA pairs from the plurality of control samples. In some of these embodiments, the term "average" as used herein can be the mean or median. In other embodiments, the cut-off is the average +/- one standard deviation, or any smaller or larger proportion of the standard deviation, for example +/- ½ standard deviation, +/- ¾ standard deviation, +/- 1.5 standard deviations, +/- 2 standard deviations, or any larger, smaller, or in-between proportion of the standard deviation.

The cut-off value can be determined without undue experimentation for any plurality of control samples from any control population by using known methods, for example by using training sets, as described in the Examples. The cut-off is established to achieve the sensitivity (SE), specificity (SP), positive predictive value (PPV) and negative predictive value (NPV) desired.

For the evaluation of these diagnostic methods (presence of tumor; presence of aggressive tumor) and prognostic methods (risk of manifesting a tumor; risk of manifesting an aggressive tumor), the categorizations interpret the results of tests in the clinical setting. The diagnostic or prognostic value of the methods can be defined by its SE, SP, PPV and NPV. Any test method will produce True Positive (TP), False Negative (FN), False Positive (FP), and True Negative (TN). The "sensitivity" of a test is the percentage of all patients with disease present or that do respond who have a positive test or (TP/TP+FN)×100%. The "specificity" of a test is the percentage of all patients without disease or who do not respond, who have a negative test or (TN/FP+TN)×100%. The "predictive value" or "PV" of a test is a measure (%) of the times that the value (positive or negative) is the true value, i.e., the percent of all positive tests that are true positives is the Positive Predictive Value (PV+) or (TP/TP+FP) x100%. The "negative predictive value" (PV) is the percentage of patients with a negative test who will not respond or (TN/FN+TN)×100%. Another measure, the "accuracy" or "efficiency" of a test, is the percentage of the times that the test give the correct answer compared to the total number of tests or (TP+TN/TP+TN+FP+FN)×100%. The "error rate" calculates from those patients predicted to respond who did not and those patients who responded that were not predicted to respond or (FP+FN/TP+TN+FP+FN)×100%. The overall test "specificity" is a measure of the accuracy of the sensitivity and specificity of a test do not change as the overall likelihood of disease changes in a population, the predictive value does change. The PV changes with a physician's clinical assessment of the presence or absence of disease or presence or absence of clinical response in a given patient.

For any given test, the TP, FN, FP and TN can be determined and adjusted by the skilled artisan without undue experimentation by, e.g., adjusting the cut-off value, adjusting the statistical significance (e.g., the P value) for making a prognostic or diagnostic determination; adjusting the accuracy of the test procedures, etc.

### B. Methods

The expression levels of the miRNAs can be determined by any means known in the art. In some embodiments, the expression levels are determined by making cDNA copies of each miRNA in each miRNA pair using reverse transcriptase-polymerase chain reaction (RT-PCR). In various embodiments, the expression ratio is further determined using real time PCR. In specific embodiments of these methods, the expression ratio is further determined using TaqMan probes. However, the methods are not limited to those embodiments and may be practiced using any appropriate method now known or later discovered for determining miRNA expression levels.

In some embodiments of the invention methods, the miRNA is amplified prior to measurement. In other embodiments, the level of those nucleic acids is measured during the amplification process. In still other methods, the nucleic acids are not amplified prior to measurement.

Many methods exist for amplifying miRNA nucleic acid sequences such as mature miRNAs, precursor miRNAs, and primary miRNAs. Suitable nucleic acid polymerization and amplification techniques include reverse transcription (RT), polymerase chain reaction (PCR), real-time PCR (quantitative PCR (q-PCR)), nucleic acid sequence-base amplification (NASBA), ligase chain reaction, multiplex ligateable probe amplification, invader technology (Third Wave), rolling circle amplification, in vitro transcription (IVT), strand displacement amplification, transcription-mediated amplification (TMA), RNA (Eberwine) amplification, and other methods that are known to persons skilled in the art. In certain embodiments, more than one amplification method is used, such as reverse transcription followed by real time quantitative PCR (qRT-PCR). In PCR and q-PCR methods, for example, a set of primers is used for each target sequence. In certain embodiments, the lengths of the primers depends on many factors, including, but not limited to, the desired hybridization temperature between the primers, the target nucleic acid sequence, and the complexity of the different target nucleic acid sequences to be amplified. In certain embodiments, a primer is about 15 to about 35 nucleotides in length. In other embodiments, a primer is equal to or fewer than 15, 20, 25, 30, or 35 nucleotides in length. In additional embodiments, a primer is at least 35 nucleotides in length.

In a further aspect, a forward primer can comprise at least one sequence that anneals to a miRNA and alternatively can comprise an additional 5' non-complementary region. In another aspect, a reverse primer can be designed to anneal to the complement of a reverse transcribed miRNA. The reverse primer may be independent of the miRNA sequence, and multiple miRNA biomarkers may be amplified using the same reverse primer. Alternatively, a reverse primer may be specific for a miRNA biomarker.

The qRT-PCR reaction may further be combined with the reverse transcription reaction by including both a reverse transcriptase and a DNA-based thermostable DNA polymerase. When two polymerases are used, a "hot start" approach may be used to maximize assay performance (U.S. Pat. Nos. 5,411,876 and 5,985,619). For example, the components for a reverse transcriptase reaction and a PCR reaction may be sequestered using one or more thermoactivation methods or chemical alteration to improve polymerization efficiency (U.S. Pat. Nos. 5,550,044, 5,413,924, and 6,403,341).

In certain embodiments, labels, dyes, or labeled probes and/or primers are used to detect amplified or unamplified miRNAs. The skilled artisan will recognize which detection methods are appropriate based on the sensitivity of the detection method and the abundance of the target. Depending on the sensitivity of the detection method and the abundance of the target, amplification may or may not be required prior to detection. One skilled in the art will recognize the detection methods where miRNA amplification is preferred.

A probe or primer may include Watson-Crick bases or modified bases. Modified bases include, but are not limited to, the AEGIS bases (from Eragen Biosciences), which have been described, e.g., in U.S. Pat. Nos. 5,432,272, 5,965,364, and 6,001,983. In certain aspects, bases are joined by a natural phosphodiester bond or a different chemical linkage. Different chemical linkages include, but are not limited to, a peptide bond or a Locked Nucleic Acid (LNA) linkage, which is described, e.g., in U.S. Pat. No. 7,060,809.

In a further aspect, oligonucleotide probes or primers present in an amplification reaction are suitable for monitoring the amount of amplification product produced as a function of time. In certain aspects, probes having different single stranded versus double stranded character are used to detect the nucleic acid. Probes include, but are not limited to, the 5'-exonuclease assay (e.g., TaqMan™) probes (see U.S. Pat. No. 5,538,848), stem-loop molecular beacons (see, e.g., U.S. Pat. Nos. 6,103,476 and 5,925,517), stemless or linear beacons (see, e.g., WO 9921881, U.S. Pat. Nos. 6,485,901 and 6,649,349), peptide nucleic acid (PNA) Molecular Beacons (see, e.g., U.S. Pat. Nos. 6,355,421 and 6,593,091), linear PNA beacons (see, e.g. U.S. Pat. No. 6,329,144), non-FRET probes (see, e.g., U.S. Pat. No. 6,150,097), Sunrise™/AmplifluorB™ probes (see, e.g., U.S. Pat. No. 6,548,250), stem-loop and duplex Scorpion™ probes (see, e.g., U.S. Pat. No. 6,589,743), bulge loop probes (see, e.g., U.S. Pat. No. 6,590,091), pseudo knot probes (see, e.g., U.S. Pat. No. 6,548,250), cyclicons (see, e.g., U.S. Pat. No. 6,383,752), MGB Eclipse™ probe (Epoch Biosciences), hairpin probes (see, e.g., U.S. Pat. No. 6,596,490), PNA light-up probes, antiprimer quench probes (Li et al., Clin. Chem. 53:624-633 (2006)), self-assembled nanoparticle probes, and ferrocene-modified probes described, for example, in U.S. Pat. No. 6,485,901.

In certain embodiments, one or more of the primers in an amplification reaction includes a label. In yet further embodiments, different probes or primers comprise detectable labels that are distinguishable from one another. In some embodiments a nucleic acid, such as the probe or primer, may be labeled with two or more distinguishable labels.

In some embodiments, the concentration of the miRNA is measured using a microarray or another support.

A "microarray" is a linear or two-dimensional or three dimensional (and solid phase) array of discrete regions, each having a defined area, formed on the surface of a solid support such as, but not limited to, glass, plastic, or synthetic membrane. The density of the discrete regions on a microarray is determined by the total numbers of immobilized polynucleotides to be detected on the surface of a single solid phase support, such as of at least about 50/cm², at least about 100/cm², or at least about 500/cm², up to about 1,000/cm² or higher. The arrays may contain less than about 500, about 1000, about 1500, about 2000, about 2500, about 3000, or more immobilized polynucleotides in total. As used herein, a DNA microarray is an array of oligonucleotide or polynucleotide probes placed on a chip or other surfaces used to hybridize to amplified or cloned polynucleotides from a sample. Since the position of each particular group of probes in the array is known, the identities of a sample polynucleotides can be determined based on their binding to a particular position in the microarray.

As an alternative to the use of a microarray, an array of any size on a support may be used in the practice of the disclosure, including an arrangement of one or more position of a two-dimensional or three-dimensional arrangement to detect expression of an miRNA.

In some aspects, a label is attached to one or more probes and has one or more of the following properties: (i) provides a detectable signal; (ii) interacts with a second label to modify the detectable signal provided by the second label, e.g., FRET (Fluorescent Resonance Energy Transfer); (iii) stabilizes hybridization, e.g., duplex formation; and (iv) provides a member of a binding complex or affinity set, e.g., affinity, antibody-antigen, ionic complexes, hapten-ligand (e.g., biotin-avidin). In still other aspects, use of labels can be accomplished using any one of a large number of known techniques employing known labels, linkages, linking groups, reagents, reaction conditions, and analysis and purification methods.

MiRNAs can be detected by direct or indirect methods. In a direct detection method, one or more miRNAs are detected by a detectable label that is linked to a nucleic acid molecule. In such methods, the miRNAs may be labeled prior to binding to the probe. Therefore, binding is detected by screening for the labeled miRNA that is bound to the probe. The probe is optionally linked to a bead in the reaction volume.

In certain embodiments, nucleic acids are detected by direct binding with a labeled probe, and the probe is subsequently detected. In one embodiment of the invention, the nucleic acids, such as amplified miRNAs, are detected using FIexMAP Microspheres (Luminex) conjugated with probes to capture the desired nucleic acids. Some methods may involve detection with polynucleotide probes modified with fluorescent labels or branched DNA (bDNA) detection, for example.

In other embodiments, nucleic acids are detected by indirect detection methods. For example, a biotinylated probe may be combined with a streptavidin-conjugated dye to detect the bound nucleic acid. The streptavidin molecule binds a biotin label on amplified miRNA, and the bound miRNA is detected by detecting the dye molecule attached to the streptavidin molecule. In one embodiment, the streptavidin-conjugated dye molecule comprises Phycolink® Streptavidin R-Phycoerythrin (PROzyme). Other conjugated dye molecules are known to persons skilled in the art.

Labels include, but are not limited to: light-emitting, light-scattering, and light-absorbing compounds which generate or quench a detectable fluorescent, chemiluminescent, or bioluminescent signal (see, e.g., Kricka, L., Nonisotopic DNA Probe Techniques, Academic Press, San Diego (1992) and Garman A., Non-Radioactive Labeling, Academic Press (1997)). Fluorescent reporter dyes useful as labels include, but are not limited to, fluoresceins (see, e.g., U.S. Pat. Nos. 5,188,934, 6,008,379, and 6,020,481), rhodamines (see, e.g., U.S. Pat. Nos. 5,366,860, 5,847,162, 5,936,087, 6,051,719, and 6,191,278), benzophenoxazines (see, e.g., U.S. Pat. No. 6,140,500), energy-transfer fluorescent dyes, comprising pairs of donors and acceptors (see, e.g., U.S. Pat. Nos. 5,863,727; 5,800,996; and 5,945,526), and cyanines (see, e.g., WO 9745539), lissamine, phycoerythrin, Cy2, Cy3, Cy3.5, Cy5, Cy5.5, Cy7, FluorX (Amersham), Alexa 350, Alexa 430, AMCA, BODIPY 630/650, BODIPY 650/665, BODIPY-FL, BODIPY-R6G, BODIPY-TMR, BODIPY-TRX, Cascade Blue, Cy3, Cy5, 6-FAM, Fluorescein Isothiocyanate, HEX, 6-JOE, Oregon Green 488, Oregon Green 500, Oregon Green 514, Pacific Blue, REG, Rhodamine Green, Rhodamine Red, Renographin, ROX, SYPRO, TAMRA, Tetramethylrhodamine, and/or Texas Red, as well as any other fluorescent moiety capable of generating a detectable signal. Examples of fluorescein dyes include, but are not limited to, 6-carboxyfluorescein; 2',4',1,4,-tetrachlorofluorescein; and 2',4',5',7',1,4-hexachlorofluorescein. In certain aspects, the fluorescent label is selected from SYBR-Green, 6-carboxyfluorescein ("FAM"), TET, ROX, VICTM, and JOE. For example, in certain embodiments, labels are different fluorophores capable of emitting light at different, spectrally-resolvable wavelengths (e.g., 4-differently colored fluorophores); certain such labeled probes are known in the art and described above, and in U.S. Pat. No. 6,140,054. A dual labeled fluorescent probe that includes a reporter fluorophore and a quencher fluorophore is used in some embodiments. It will be appreciated that pairs of fluorophores are chosen that have distinct emission spectra so that they can be easily distinguished.

In still a further aspect, labels are hybridization-stabilizing moieties which serve to enhance, stabilize, or influence hybridization of duplexes, e.g., intercalators and intercalating dyes (including, but not limited to, ethidium bromide and SYBR-Green), minor-groove binders, and cross-linking functional groups (see, e.g., Blackburn et al., eds. "DNA and RNA Structure" in Nucleic Acids in Chemistry and Biology (1996)).

In further aspects, methods relying on hybridization and/or ligation to quantify miRNAs may be used, including oligonucleotide ligation (OLA) methods and methods that allow a distinguishable probe that hybridizes to the target nucleic acid sequence to be separated from an unbound probe. As an example, HARP-like probes, as disclosed in U.S. Publication No. 2006/0078894 may be used to measure the quantity of miRNAs.

In an additional embodiment of the method, a probe ligation reaction may be used to quantify miRNAs. In a Multiplex Ligation-dependent Probe Amplification (MLPA) technique (Schouten et al., Nucleic Acids Research 30:e57 (2002)), pairs of probes which hybridize immediately adjacent to each other on the target nucleic acid are ligated to each other only in the presence of the target nucleic acid. In some aspects, MLPA probes have flanking PCR primer binding sites. MLPA probes can only be amplified if they have been ligated, thus allowing for detection and quantification of miRNA biomarkers.

### C. Detection of Hemolysis

Hemolysis of red blood cells and platelets release miRNAs that can interfere with the above methods (Kirschner et al., PLoS One 6:e24145, 2011; Pritchard et al., Cancer Prev Res (Phila) 5:492-497, 2012). Thus, in some embodiments of the above methods, hemolysis is determined in the biological sample and the sample is not subjected to the method if hemolysis is determined.

Hemolysis can be determined in the subject samples by any means known in the art. In some embodiments, hemolysis is determined spectrophotometrically, e.g., by measuring the absorbance at different wavelengths (414nm, 541nm, 576nm) to identify the presence and amount of free hemoglobin in the sample (Kirschner et al., 2011).

In other embodiments, hemolysis is determined by analyzing expression levels of hemolysis-related miRNAs that are upregulated in hemolyzed samples. In some of these embodiments, any of the miRNAs miR-451, miR-486-5p, miR-16, miR-92a or miR-140-3p, which are significantly upregulated in hemolysed samples vs. non-hemolysed samples, are utilized. In various aspects of these embodiments, the hemolysis-related miRNAs are miR-451, miR-486-5p, miR-16, and miR-92a.

In further embodiments, expression levels of a plurality of normalizing miRNAs that are not upregulated in hemolysed samples are determined, and used to normalize the expression levels of the hemolysis-related miRNAs. These normalizing miRNAs can be used in ratios with hemolysis-related miRNAs to normalize the determined expression levels of the latter miRNAs. In some of these embodiments, the normalizing miRNAs comprise miR-126, miR-15b, miR-221 and miR-30b.

### "Hemolysis" Signature

In some embodiments, hemolysis is further determined by:
(a) determining the expression ratio of each of 16 miRNA pairs consisting of each of miR-451, miR-486-5p, miR-16, and miR-92a paired with each of miR-126, miR-15b, miR-221 and miR-30b (i.e., miR-451/miR-126, miR-451/miR-15b, miR-451/miR-221, miR-451/miR-30b, miR-486-5p/miR-126, miR-486-5p/miR-15b, miR-486-5p/miR-221, miR-486-5p/miR-30b, miR-16/miR-126, miR-16/miR-15b, miR-16/miR-221, miR-16/miR-30b, miR-92a/miR-126, miR-92a/miR-15b, miR-92a/miR-221, and miR-92a/miR-30b or the inverse ratios thereof) in the sample;
(b) comparing each of the 16 expression ratios from step (a) with a cut-off value determined for each expression ratio from the average ratio of a plurality of corresponding miRNA pairs from a plurality of control samples;
(c) for each of the 16 miRNA pairs, assign a positive score for the expression ratio in step (a) if the ratio exceeds the cut-off value in step (b), or assign a non-positive score for the expression ratio in step (a) if the ratio does not exceed the cut-off value in step (b); and
(d) categorize the sample as (i) having hemolysis if eight or more out of the 16 ratios exceed the cutoff value, or (ii) not having hemolysis if fewer than eight out of the 16 ratios exceed the cutoff value.

As with the diagnostic and prognostic assays described above, the cut-off value of this hemolysis assay depends on the control population, e.g., the cut-off where a plurality of unhemolyzed serum samples are used as the control samples will be different than the cut-off where a plurality of hemolyzed serum samples are used as the control samples. See Examples. Additionally, the cut-off can be adjusted, e.g., to achieve desired SE, SP, PPV and NPV values. In some embodiments where unhemolyzed serum samples are used as the control samples the cut-off will be the average (mean or median) ratio of the plurality of control samples. Optionally, this cut-off will have a +/- proportion of a standard deviation to account for differences in variability among the samples used to determine the cut-off value.

In various embodiments, any of the hemolysis assays using miRNA expression levels described above is also combined with another hemolysis assay, for example the spectrophotometric assay described in Kirschner et al., 2011.

### D. Combinations of Diagnosis

As discussed in the Examples, combining the MSC signature described therein with low-dose computed tomography (LDCT) allows the detection of additional cancers undetected by LDCT, raising the screening sensitivity from 87% for MSC alone and 84% for LDCT alone to 98% when LDCT is combined with MSC. In combination with cancer screening assays other than LDCT, the diagnostic and prognostic methods above would be expected to show similar improvements in diagnostic sensitivity.

Health care costs of LDCT screening and associated follow-up procedures are significant. A recent budget impact model considering LDCT as widely adopted in the United States indicated that LDCT screening would avoid up to 8100 premature lung cancer deaths at a 75% screening rate with an additional screening cost of $240,000 to avoid one lung cancer death (Goulart et al., 2012). Complementing LDCT screening with a non-invasive biomarker test, by reducing down-stream costs, might increase the number of individuals enrolled in LDCT screening (Peres, J Natl Cancer Inst 105:1-2, 2013).

Thus, any of the above-described diagnostic or prognostic methods can be combined with a screening of the subject for lung cancer using a system that does not comprise analysis of miRNA expression. The system may be used prior to, concurrently, or after the miRNA expression analysis. Any system now known or later discovered would be expected to benefit from additional analysis with MSC. Non-limiting examples of such systems include a blood test, an x-ray, computed tomography, positron emission tomography, thoracentesis, bronchoscopy, fine-needle aspiration, thoracoscopy, thoracotomy, or mediastinoscopy. In some embodiments, the system is low-dose computed tomography (LDCT).

### E. Monitoring

Any of the above methods can be used for monitoring the subject for presence of lung cancer and/or risk of developing lung cancer. A subject is monitored when any of the above methods are performed on at least two different biological samples that were taken from the subject at different times. In some embodiments, at least one of the at least two different biological samples is taken from the subject after the subject has been treated for lung cancer. Such monitoring evaluates recurrence or risk of recurrence of the lung cancer.

### F. Treatment

The above methods can be utilized to select treatment options. As used herein, the term "treat" is meant to describe administering an agent to eliminate or reduce in severity a sign or symptom of lung cancer. Alternatively, or in addition, a disorder which can occur in multiple locations, is treated if therapy is applied to that disorder in at least one of multiple locations.

According to one embodiment, the above methods can be utilized to select treatment options is where the subject is not treated for lung cancer if a negative presence or risk is determined, or if a low risk is determined in the combined method for predicting the risk of developing or having a tumor. In another embodiment, a subject is treated for lung cancer if a positive presence or risk is determined, or if an intermediate or high risk in the combined method is determined.

Thus, also provided herewith is a method of establishing lung cancer treatment options for a subject. The method comprises testing the subject using of the diagnostic and/or prognostic methods described above, and determining treatment options according to the results of the method. The method may further comprise administering a treatment to the subject in need thereof.

### G. MiRNAs

Overall, 24 miRNAs may compose the signature of Presence of a Pulmonary Tumor (PD), the signature of Presence of an Aggressive Pulmonary Tumor (PAD), the signature of Risk of Manifesting a Pulmonary Tumor (RD), and the signature of Risk of Manifesting an Aggressive Pulmonary Tumor (RAD). Table 1 recites those 24 miRNAs which may be present in each signature.

**Table 1**

| **miRNA** |
|---|
| hsa-miR-16 |
| hsa-miR-17 |
| hsa-miR-21 |
| hsa-miR-101 |
| hsa-miR-126 |
| hsa-miR-145 |
| hsa-miR-197 |
| hsa-miR-221 |
| hsa-miR-320 |
| hsa-miR-451 |
| hsa-miR-660 |
| hsa-miR-106a |
| hsa-miR-133a |
| hsa-miR-140-3p |
| hsa-miR-140-5p |
| hsa-miR-142-3p |
| hsa-miR-148a |
| hsa-miR-15b |
| hsa-miR-19b |
| hsa-miR-28-3p |
| hsa-miR-30b |
| hsa-miR-30c |
| hsa-miR-486-5p |
| hsa-miR-92a |

Table 2 provides a summary of the miRNA for use in all aspects of the present invention.

**Table 2**

| **miRNA Name** | **Sequence** |
|---|---|
| hsa-miR-7-2 (pre-miR) | CUGGAUACAGAGUGGACCGGCUGGCCCCAUCUGGAAGACUAGU GAUUUUGUUGUUGUCUUACUGCGCUCAACAACAAAUCCCAGUC UACCUAAUGGUGCCAGCCAUCGCA  (SEQ ID NO:1) |
| hsa-miR-7-2-5p (mature miR 5' arm) | UGGAAGACUAGUGAUUUUGUUGU (SEQ ID NO:2) |
| hsa-miR-7-2-3p (mature miR 3' arm) | CAACAAAUCCCAGUCUACCUAA (SEQ ID NO:3) |
| hsa-miR-15b (pre-miR) | UUGAGGCCUUAAAGUACUGUAGCAGCACAUCAUGGUUUACAUG CUACAGUCAAGAUGCGAAUCAUUAUUUGCUGCUCUAGAAAUUU AAGGAAAUUCAU (SEQ ID NO:4) |
| hsa-miR-15b-5p (mature miR 5'arm) | UAGCAGCACAUCAUGGUUUACA (SEQ ID NO:5) |
| hsa-miR-15b-3p (mature miR 3'arm) | CGAAUCAUUAUUUGCUGCUCUA (SEQ ID NO:6) |
| hsa-miR-16-1 (pre-miR from Chr.13) | GUCAGCAGUGCCUUAGCAGCACGUAAAUAUUGGCGUUAAGAUU CUAAAAUUAUCUCCAGUAUUAACUGUGCUGCUGAAGUAAGGUU GAC (SEQ ID NO:7) |
| hsa-miR-16-2 (pre-miR from Chr.3) | GUUCCACUCUAGCAGCACGUAAAUAUUGGCGUAGUGAAAUAUA UAUUAAACACCAAUAUUACUGUGCUGCUUUAGUGUGAC (SEQ ID NO:8) |
| hsa-miR-16-5p (mature miR 5' arm) | UAGCAGCACGUAAAUAUUGGCG (SEQ ID NO:9) |
| hsa-miR-16-3p (mature miR 3'arm) | CCAAUAUUACUGUGCUGCUUUA (SEQ ID NO:10) |
| hsa-miR-17 (pre-miR) | GUCAGAAUAAUGUCAAAGUGCUUACAGUGCAGGUAGUGAUAUG UGCAUCUACUGCAGUGAAGGCACUUGUAGCAUUAUGGUGAC (SEQ ID NO:11) |
| hsa-miR-17-5p (mature miR 5'arm) | CAAAGUGCUUACAGUGCAGGUAG (SEQ ID NO:12) |
| hsa-miR-17-3p (mature miR 3'arm) | ACUGCAGUGAAGGCACUUGUAG (SEQ ID NO:13) |
| hsa-miR-19b-1 (pre-miR from Chr. 13) | CACUGUUCUAUGGUUAGUUUUGCAGGUUGCAUCCAGCUGUGU GAUAUUCUGCUGUGCAAAUCCAUGCAAAACUGACUGUGGUAGU G (SEQ ID NO:14) |
| hsa-miR-19b-1-5p (mature miR 5'arm from Chr. 13) | AGUUUUGCAGGUUUGCAUCCAGC (SEQ ID NO:15) |
| hsa-miR-19b-2 (pre-miR from Chr. X) | ACAUUGCUACUUACAAUUAGUUUUGCAGGUUUGCAUUUCAGCG UAUAUAUGUAUAUGUGGCUGUGCAAAUCCAUGCAAAACUGAUU GUGAUAAUGU (SEQ ID NO:16) |
| hsa-miR-19b-2-5p (mature miR 5' arm from Chr. X) | AGUUUUGCAGGUUUGCAUUUCA (SEQ ID NO:17) |
| hsa-miR-19b-3p (mature miR 3' arm from Chr. 13 or X) | UGUGCAAAUCCAUGCAAAACUGA (SEQ ID NO:18) |
| hsa-miR-21 (pre-miR) | UGUCGGGUAGCUUAUCAGACUGAUGUUGACUGUUGAAUCUCAU GGCAACACCAGUCGAUGGGCUGUCUGACA (SEQ ID NO:19) |
| hsa-miR-21-5p (mature miR 5' arm) | UAGCUUAUCAGACUGAUGUUGA (SEQ ID NO:20) |
| hsa-miR-21-3p (mature miR 3' arm) | CAACACCAGUCGAUGGGCUGU (SEQ ID NO:21) |
| hsa-miR-28 (pre-miR) | GGUCCUUGCCCUCAAGGAGCUCACAGUCUAUUGAGUUACCUUU CUGACUUUCCCACUAGAUUGUGAGCUCCUGGAGGGCAGGCACU (SEQ ID NO:22) |
| hsa-miR-28-5p (mature miR 5' arm) | AAGGAGCUCACAGUCUAUUGAG (SEQ ID NO:23) |
| hsa-miR-28-3p (mature miR 3' arm) | CACUAGAUUGUGAGCUCCUGGA (SEQ ID NO:24) |
| hsa-miR-30a (pre-miR) | GCGACUGUAAACAUCCUCGACUGGAAGCUGUGAAGCCACAGAU GGGCUUUCAGUCGGAUGUUUGCAGCUGC (SEQ ID NO:25) |
| hsa-miR-30a-5p (mature miR 5' arm) | UGUAAACAUCCUCGACUGGAAG (SEQ ID NO:26) |
| hsa-miR-30a-3p (mature miR 3' arm) | CUUUCAGUCGGAUGUUUGCAGC (SEQ ID NO:27) |
| hsa-miR-30b (pre-miR) | ACCAAGUUUCAGUUCAUGUAAACAUCCUACACUCAGCUGUAAU ACAUGGAUUGGCUGGGAGGUGGAUGUUUACUUCAGCUGACUUG GA (SEQ ID NO:28) |
| hsa-miR-30b-5p (mature miR 5' arm) | UGUAAACAUCCUACACUCAGCU (SEQ ID NO:29) |
| hsa-miR-30b-3p (mature miR 3' arm) | CUGGGAGGUGGAUGUUUACUUC (SEQ ID NO:30) |
| hsa-miR-30c-1 (pre-miR from Chr. 1) | ACCAUGCUGUAGUGUGUGUAAACAUCCUACACUCUCAGCUGUG AGCUCAAGGUGGCUGGGAGAGGGUUGUUUACUCCUUCUGCCAU GGA (SEQ ID NO:31) |
| hsa-miR-30c-1-3p (mature miR 3' arm from Chr. 1) | CUGGGAGAGGGUUGUUUACUCC (SEQ ID NO:32) |
| hsa-miR-30c-2 (pre-miR from Chr. 6) | AGAUACUGUAAACAUCCUACACUCUCAGCUGUGGAAAGUAAGA AAGCUGGGAGAAGGCUGUUUACUCUUUCU (SEQ ID NO:33) |
| hsa-miR-30c-5p (mature miR 5' arm from Chr. 1 or 6) | UGUAAACAUCCUACACUCUCAGC (SEQ ID NO:34) |
| hsa-miR-30c-2-3p (mature miR 3' arm from Chr. 6) | CUGGGAGAAGGCUGUUUACUCU (SEQ ID NO:35) |
| hsa-miR-30d (pre-miR) | GUUGUUGUAAACAUCCCCGACUGGAAGCUGUAAGACACAGCUA AGCUUUCAGUCAGAUGUUUGCUGCUAC (SEQ ID NO:36) |
| hsa-miR-30d-5p (mature miR 5' arm) | UGUAAACAUCCCCGACUGGAAG (SEQ ID NO:37) |
| hsa-miR-30d-3p (mature miR 3' arm) | CUUUCAGUCAGAUGUUUGCUGC (SEQ ID NO:38) |
| hsa-miR-34b (pre-miR) | GUGCUCGGUUUGUAGGCAGUGUCAUUAGCUGAUUGUACUGUGG UGGUUACAAUCACUAACUCCACUGCCAUCAAAACAAGGCAC (SEQ ID NO:39) |
| hsa-miR-34b-5p (mature miR 5' arm) | UAGGCAGUGUCAUUAGCUGAUUG (SEQ ID NO:40) |
| hsa-miR-34b-3p (mature miR 3' arm) | CAAUCACUAACUCCACUGCCAU (SEQ ID NO:41) |
| hsa-mirR-92a-1 (pre-miR from Chr. 13) | CUUUCUACACAGGUUGGGAUCGGUUGCAAUGCUGUGUUUCUGU AUGGUAUUGCACUUGUCCCGGCCUGUUGAGUUUGG (SEQ ID NO:42) |
| hsa-miR-92a-1-5p (mature miR 5' arm from Chr. 13) | AGGUUGGGAUCGGUUGCAAUGCU (SEQ ID NO:43) |
| hsa-miR-92a-3p (mature miR 3' arm from Chr. 13 or X) | UAUUGCACUUGUCCCGGCCUGU (SEQ ID NO:44) |
| hsa-miR-92a-2 (pre-miR from Chr. X) | UCAUCCCUGGGUGGGGAUUUGUUGCAUUACUUGUGUUCUAUAU AAAGUAUUGCACUUGUCCCGGCCUGUGGAAGA (SEQ ID NO:45) |
| hsa-miR-92a-2-5p (mature miR 5' arm from Chr. X) | GGGUGGGGAUUUGUUGCAUUAC (SEQ ID NO:46) |
| hsa-miR-101-1 (pre-miR from Chr. 1) | UGCCCUGGCUCAGUUAUCACAGUGCUGAUGCUGUCUAUUCUAA AGGUACAGUACUGUGAUAACUGAAGGAUGGCA (SEQ ID NO:47) |
| hsa-miR-101-5p (mature miR 5' arm from Chr. 1 or 9) | CAGUUAUCACAGUGCUGAUGCU (SEQ ID NO:48) |
| hsa-miR-101-1-3p (mature miR 3' arm from Chr. 1) | UACAGUACUGUGAUAACUGAA (SEQ ID NO:49) |
| hsa-miR-101-2 (pre-miR from Chr. 9) | ACUGUCCUUUUUCGGUUAUCAUGGUACCGAUGCUGUAUAUCUG AAAGGUACAGUACUGUGAUAACUGAAGAAUGGUGGU (SEQ ID NO:50) |
| hsa-miR-101-2-3p (mature miR 3' arm from Chr. 9) | UACAGUACUGUGAUAACUGAA (SEQ ID NO:51) |
| hsa-miR-106a (pre-miR) | CCUUGGCCAUGUAAAAGUGCUUACAGUGCAGGUAGCUUUUUGA GAUCUACUGCAAUGUAAGCACUUCUUACAUUACCAUGG (SEQ ID NO:52) |
| hsa-miR-106a-5p (mature miR 5' arm) | AAAAGUGCUUACAGUGCAGGUAG (SEQ ID NO:53) |
| hsa-miR 106a-3p (mature miR 3' arm) | CUGCAAUGUAAGCACUUCUUAC (SEQ ID NO:54) |
| hsa-miR-126 (pre-miR) | CGCUGGCGACGGACAUUAUUACUUUUGGUACGCGCUGUGACA CUUCAAACUCGUACCGUGAGUAAUAAUGCGCCGUCCACGGCA (SEQ ID NO:55) |
| hsa-miR-126-5p (mature miR 5' arm) | CAUUAUUACUUUUGGUACGCG (SEQ ID NO:56) |
| hsa-miR-126-3p (mature miR 3' arm) | UCGUACCGUGAGUAAUAAUGCG (SEQ ID NO:57) |
| hsa-miR-133a-1 (pre-miR from Chr. 18) | ACAAUGCUUUGCUAGAGCUGGUAAAAUGGAACCAAAUCGCCUC UUCAAUGGAUUUGGUCCCCUUCAACCAGCUGUAGCUAUGCAUU GA (SEQ ID NO:58) |
| hsa-miR 133a-2 (pre-miR from Chr. 20) | GGGAGCCAAAUGCUUUGCUAGAGCUGGUAAAAUGGAACCAAAU CGACUGUCCAAUGGAUUUGGUCCCCUUCAACCAGCUGUAGCUG UGCAUUGAUGGCGCCG (SEQ ID NO:59) |
| hsa-miR-133a (mature miR 3' arm from Chr. 18 or 20) | UUUGGUCCCCUUCAACCAGCUG (SEQ ID NO:60) |
| hsa-miR-140 (pre-miR) | UGUGUCUCUCUCUGUGUCCUGCCAGUGGUUUUACCCUAUGGUA GGUUACGUCAUGCUGUUCUACCACAGGGUAGAACCACGGACAG GAUACCGGGGCACC (SEQ ID NO:61) |
| hsa-miR-140-5p (mature miR 5' arm) | CAGUGGUUUUACCCUAUGGUAG (SEQ ID NO:62) |
| hsa-miR-140-3p (mature miR 3' arm) | UACCACAGGGUAGAACCACGG (SEQ ID NO:63) |
| hsa-miR-142 (pre-miR) | GACAGUGCAGUCACCCAUAAAGUAGAAAGCACUACUAACAGCA CUGGAGGGUGUAGUGUUUCCUACUUUAUGGAUGAGUGUACUGU G (SEQ ID NO:64) |
| hsa-miR-142-5p (mature miR 5' arm) | CAUAAAGUAGAAAGCACUACU (SEQ ID NO:65) |
| hsa-miR-142-3p (mature miR 3' arm) | UGUAGUGUUUCCUACUUUAUGGA (SEQ ID NO:66) |
| hsa-miR-144 (pre-miR) | UGGGGCCCUGGCUGGGAUAUCAUCAUAUACUGUAAGUUUGCGA UGAGACACUACAGUAUAGAUGAUGUACUAGUCCGGGCACCCCC (SEQ ID NO:67) |
| hsa-miR-144-5p (mature miR 5' arm) | GGAUAUCAUCAUAUACUGUAAG (SEQ ID NO:68) |
| hsa-miR-144-3p (mature miR 3' arm) | UACAGUAUAGAUGAUGUACU (SEQ ID NO:69) |
| hsa-miR-145 (pre-miR) | CACCUUGUCCUCACGGUCCAGUUUUCCCAGGAAUCCCUUAGAU GCUAAGAUGGGAUUCCUGGAAAUACUGUUCUUGAGGUCAUGG UU (SEQ ID NO:70) |
| hsa-miR-145-5p (mature miR 5' arm) | GUCCAGUUUUCCCAGGAAUCCCU (SEQ ID NO:71) |
| hsa-miR-145-3p (mature miR 3'arm) | GGAUUCCUGGAAAUACUGUUCU (SEQ ID NO:72) |
| hsa-miR-148a (pre-miR) | GAGGCAAAGUUCUGAGACACUCCGACUCUGAGUAUGAUAGAAG UCAGUGCACUACAGAACUUUGUCUC (SEQ ID NO:73) |
| hsa-miR-148a-5p (mature miR 5' arm) | AAAGUUCUGAGACACUCCGACU (SEQ ID NO:74) |
| hsa-miR-148a-3p (mature miR 3' arm) | UCAGUGCACUACAGAACUUUGU (SEQ ID NO:75) |
| hsa-miR-197 (pre-miR) | GGCUGUGCCGGGUAGAGAGGGCAGUGGGAGGUAAGAGCUCUUC ACCCUUCACCACCUUCUCCACCCAGCAUGGCC (SEQ ID NO:76) |
| hsa-miR-197-5p (mature miR 5' arm) | CGGGUAGAGAGGGCAGUGGGAGG (SEQ ID NO:77) |
| hsa-miR-197-3p (mature miR 3' arm) | UUCACCACCUUCUCCACCCAGC (SEQ ID NO:78) |
| hsa-miR-200b (pre miR) | CCAGCUCGGGCAGCCGUGGCCAUCUUACUGGGCAGCAUUGGAU GGAGUCAGGUCUCUAAUACUGCCUGGUAAUGAUGACGGCGGAG CCCUGCACG (SEQ ID NO:79) |
| hsa-miR-200b-5p (mature miR 5' arm) | CAUCUUACUGGGCAGCAUUGGA (SEQ ID NO:80) |
| hsa-miR-200b-3p (mature miR 3' arm) | UAAUACUGCCUGGUAAUGAUGA (SEQ ID NO:81) |
| hsa-miR-205 (pre-miR) | AAAGAUCCUCAGACAAUCCAUGUGCUUCUCUUGUCCUUCAUUC CACCGGAGUCUGUCUCAUACCCAACCAGAUUUCAGUGGAGUGA AGUUCAGGAGGCAUGGAGCUGACA  (SEQ ID NO:82) |
| hsa-miR-205-5p (mature miR 5' arm) | UCCUUCAUUCCACCGGAGUCUG (SEQ ID NO:83) |
| hsa-miR-205-3p (mature miR 3' arm) | GAUUUCAGUGGAGUGAAGUUC (SEQ ID NO:84) |
| hsa-miR-210 (pre-miR) | ACCCGGCAGUGCCUCCAGGCGCAGGGCAGCCCCUGCCCACCGC ACACUGCGCUGCCCCAGACCCACUGUGCGUGUGACAGCGGCUG AUCUGUGCCUGGGCAGCGCGACCC (SEQ ID NO:85) |
| hsa-miR-210 (mature miR) | CUGUGCGUGUGACAGCGGCUGA (SEQ ID NO:86) |
| hsa-miR-219-1 (pre-miR) | CCGCCCCGGGCCGCGGCUCCUGAUUGUCCAAACGCAAUUCUCG AGUCUAUGGCUCCGGCCGAGAGUUGAGUCUGGACGUCCCGAGC CGCCGCCCCCAAACCUCGAGCGGG (SEQ ID NO:87) |
| hsa-miR-219-1-5p (mature miR 5' arm) | UGAUUGUCCAAACGCAAUUCU (SEQ ID NO:88) |
| hsa-miR-219-1-3p (mature miR 3' arm) | AGAGUUGAGUCUGGACGUCCCG (SEQ ID NO:89) |
| hsa-miR-221 (pre-miR) | UGAACAUCCAGGUCUGGGGCAUGAACCUGGCAUACAAUGUAGA UUUCUGUGUUCGUUAGGCAACAGCUACAUUGUCUGCUGGGUUU CAGGCUACCUGGAAACAUGUUCUC (SEQ ID NO:90) |
| hsa-miR-221-5p (mature miR 5' arm) | ACCUGGCAUACAAUGUAGAUUU (SEQ ID NO:91) |
| hsa-miR-221-3p (mature miR 3' arm) | AGCUACAUUGUCUGCUGGGUUUC (SEQ ID NO:92) |
| hsa-miR-320a (pre-miR) | GCUUCGCUCCCCUCCGCCUUCUCUUCCCGGUUCUUCCCGGAGU CGGGAAAAGCUGGGUUGAGAGGGCGAAAAGGAUGAGGU (SEQ ID NO:93) |
| hsa-miR-320a (mature miR) | AAAAGCUGGGUUGAGAGGGCGA (SEQ ID NO:94) |
| hsa-miR-320b-1 (pre-miR from Chr. 1:117214371-117214449) | AAUUAAUCCCUCUCUUUCUAGUUCUUCCUAGAGUGAGGAAAAG CUGGGUUGAGAGGGCAAACAAAUUAACUAAUUAAUU (SEQ ID NO:95) |
| hsa-miR-320b-2 (pre-miR from Chr. 1:224444706-224444843) | UGUUAUUUUUGUCUUCUACCUAAGAAUUCUGUCUCUUAGGCU UUCUCUUCCCAGAUUUCCCAAAGUUGGAAAAGCUGGGUUGAG AGGGCAAAAGGAAAAAAAAAGAAUUCUGUCUCUGACAUAAUUA GAUAGGGAA (SEQ ID NO:96) |
| hsa-miR-320b (mature miR from Chr. 1) | AAAAGCUGGGUUGAGAGGGCAA (SEQ ID NO:97) |
| hsa-miR-320c-1 (pre-miR from Chr. 18:19263471-19263558) | UUUGCAUUAAAAAUGAGGCCUUCUCUUCCCAGUUCUUCCCAGA GUCAGGAAAAGCUGGUUGAGAGGUAGAAAAAAAUGAUGUA GG (SEQ ID NO:98) |
| hsa-miR-320c-2 (pre-miR from Chr. 18-21901650-21901699) | CUUCUCUUUCCAGUUCUUCCCAGAAUUGGGAAAAGCUGGGUUG AGAGGGU (SEQ ID NO:99) |
| hsa-miR-320-c (mature miR from either Chr 18 loci) | AAAAGCUGGGUUGAGAGGGU (SEQ ID NO:100) |
| hsa-miR-320d-1 (pre-miR from Chr. 13) | UUCUCGUCCCAGUUCUUCCCAAAGUUGAGAAAAGCUGGGUUGA GAGGA (SEQ ID NO:101) |
| hsa-miR-320d-2 (pre-miR from Chr. X) | UUCUCUUCCCAGUUCUUCUUGGAGUCAGGAAAAGCUGGGUUGA GAGGA (SEQ ID NO:102) |
| hsa-miR-320d (mature miR from Chr. 13 or X) | AAAAGCUGGGUUGAGAGGA (SEQ ID NO:103) |
| hsa-miR-320e (pre-miR) | GCCUUCUCUUCCCAGUUCUUCCUGGAGUCGGGGAAAAGCUGGG UUGAGAAGGU (SEQ ID NO:104) |
| hsa-miR-320e (mature miR) | AAAGCUGGGUUGAGAAGG (SEQ ID NO:105) |
| hsa-miR-324 (pre-miR) | CUGACUAUGCCUCCCCGCAUCCCCUAGGGCAUUGGUGUAAAGC UGGAGACCCACUGCCCCAGGUGCUGCUGGGGGUUGUAGUC (SEQ ID NO:106) |
| hsa-miR-324 (mature miR 5' arm) | CGCAUCCCCUAGGGCAUUGGUGU (SEQ ID NO:107) |
| hsa-miR-324 (mature miR 3' arm) | ACUGCCCCAGGUGCUGCUGG (SEQ ID NO:108) |
| hsa-miR-429 (pre-miR) | CGCCGGCCGAUGGGCGUCUUACCAGACAUGGUUAGACCUGGCC CUCUGUCUAAUACUGUCUGGUAAAACCGUCCAUCCGCUGC (SEQ ID NO:109) |
| hsa-miR-429 (mature miR) | UAAUACUGUCUGGUAAAACCGU (SEQ ID NO:110) |
| hsa-miR-451a (pre-miR) | CUUGGGAAUGGCAAGGAAACCGUUACCAUUACUGAGUUUAGUA AUGGUAAUGGUUCUCUUGCUAUACCCAGA (SEQ ID NO:111) |
| hsa-miR-451a (mature miR) | AAACCGUUACCAUUACUGAGUU (SEQ ID NO:112) |
| hsa-miR-451b (pre-miR) | UGGGUAUAGCAAGAGAACCAUUACCAUUACUAAACUCAGUAAU GGUAACGGUUUCCUUGCCAUUCCCA (SEQ ID NO:113) |
| hsa-miR-451b (mature miR) | UAGCAAGAGAACCAUUACCAUU (SEQ ID NO:114) |
| hsa-miR-486 (pre-miRNA) | GCAUCCUGUACUGAGCUGCCCCGAGGCCCUUCAUGCUGCCCAG CUCGGGGCAGCUCAGUACAGGAUAC (SEQ ID NO:115) |
| hsa-miR-486-5p (mature miR 5' arm) | UCCUGUACUGAGCUGCCCCGAG (SEQ ID NO:116) |
| hsa-miR-486-3p (mature miR 3' arm) | CGGGGCAGCUCAGUACAGGAU (SEQ ID NO:117) |
| hsa-miR-518e (pre-miR) | UCUCAGGCUGUGACCCUCUAGAGGGAAGCGCUUUCUGUUGGCU AAAAGAAAAGAAAGCGCUUCCCUCAGAGUGUUAACGCUUUGA GA (SEQ ID NO:118) |
| hsa-miR-518e 5p (mature miR 5' arm) | CUCUAGAGGGAAGCGCUUUCUG (SEQ ID NO:119) |
| hsa-miR-518e-3p (mature miR 3' arm) | AAAGCGCUUCCCUUCAGAGUG (SEQ ID NO:120) |
| hsa-miR-660 (pre-miR) | CUGCUCCUUCUCCCAUACCCAUUGCAUAUCGGAGUUGUGAAUU CUCAAAACACCUCCUGUGUGCAUGGAUUACAGGAGGGUGAGCC UUGUCAUCGUG (SEQ ID NO:121) |
| hsa-miR-660-5p (mature miR 5' arm) | UACCCAUUGCAUAUCGGAGUUG (SEQ ID NO:122) |
| hsa-miR-660-3p (mature miR 3' arm) | ACCUCCUGUGUGCAUGGAUUA (SEQ ID NO:123) |

The following examples are provided to better illustrate the claimed invention and are not to be interpreted as limiting the scope of the invention. To the extent that specific materials are mentioned, it is merely for purposes of illustration and is not intended to limit the invention. One skilled in the art may develop equivalent means or reactants without the exercise of inventive capacity and without departing from the scope of the invention.

### EXAMPLES

The following examples relate to the clinical utility of a plasma-based microRNA signature classifier within computed tomography lung cancer screening, also referred to as the Correlative MILD Trial Study. Provided are results from a validation study to determine the diagnostic performance of a pre-specified miRNA signature classifier (MSC) algorithm in 939 subjects retrospectively evaluated in samples prospectively collected from smoker subjects within the randomized Multicentre Italian Lung Detection (MILD) clinical trial of LDCT versus observation (Pastorino et al., 2012). We demonstrate that MSC has significant diagnostic and prognostic performance.

### Example 1: Methods

**A. Study population.** The Multicentre Italian Lung Detection (MILD) trial, a randomized prospective clinical trial, was launched in 2005 and enrolled at the Istituto Nazionale dei Tumori of Milan 4,099 current or former smokers, at least 50 years old and without history of cancer within the prior five years: 2,376 (58%) were randomized to the LDCT arms (1190 annual, 1186 biennial LDCT) and 1,723 (42%) to the observational arm (Pastorino et al., 2012). At the time of enrollment (baseline) and of each annual or biennial recall of all volunteers of the trial, whole blood was collected as described (Boeri et al., 2011) according to the Internal Review and the Ethics Boards of the Istituto Nazionale dei Tumori of Milan.

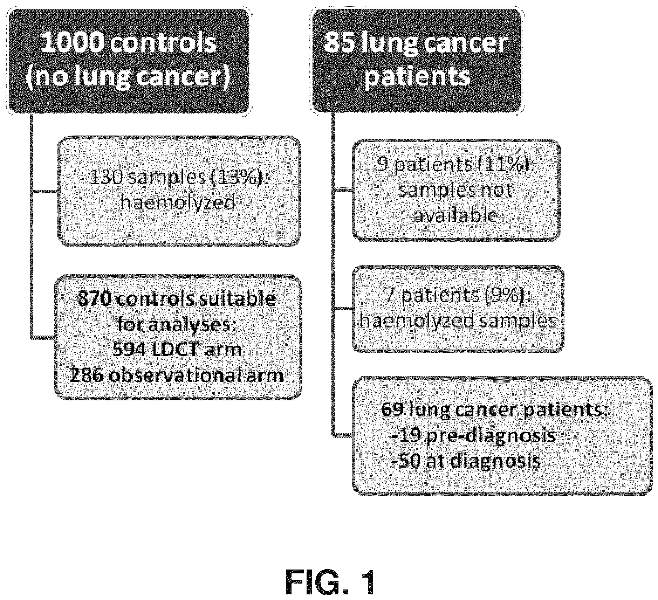

For this study, 1,000 consecutive plasma samples collected from June 2009 to July 2010 among lung cancer-free individuals enrolled in the trial were used to determine the specificity of the MSC. Plasma samples were first assayed for hemolysis (see below) to remove samples from patients that were potentially contaminated by red blood cells miRNAs (Kirschner et al., 2011; Pritchard et al., 2012). Of the 1000 samples, 130 were not evaluable because of hemolysis. Of the remaining 870 subjects, 594 (68%) belonged to the LDCT arms and 276 (32%) to the observational arm. To obtain a cohort for determining the sensitivity performance of MSC, plasma samples from almost all patients with lung cancer diagnosed by September 2012 were obtained (N=85). We favored measuring the Negative Predictive Value (NPV) in a large, unselected series of subjects instead of matching cases and controls, which would have greatly reduced the number of controls and the power of the study. For 69 of these 85 patients, at least one evaluable sample was collected. For all patients we considered the sample closest to LDCT examination resulting in cancer diagnosis. Specifically, a sample at-diagnosis was available for 50 patients and a pre-disease sample for 19 patients (FIG. 1). The pre-disease samples were collected from 8 to 35 months before lung cancer detection with a median lag time of 18 months.

**B. MicroRNA profiling.** Total RNA was extracted from 200 µl plasma samples with the mirVana PARISKit (Life Technologies, Ambion) and eluted in 50 µl of buffer. miRNA expression was determined in 3 µl of eluted RNA using the Multiplex Pools Protocol on custom-made microfluidics card (Life Technologies, Applied Biosystems) containing the 24 miRNAs spotted on duplicates. For each sample, Ct's of individual miRNAs was determined using ViiA7 software (Life Technologies, Applied Biosystems) with a threshold of 0.15 and an automatic baseline. For input into the MSC, the average of the duplicate readings of pre-defined miRNA ratios was calculated as previously described (Boeri et al., 2011).

**C. Statistical analysis.** MSC risk scores blinded to clinical outcome for individual subjects was submitted to an independent research center, the Istituto Mario Negri of Milan, and data analysis was completed as per a pre-specified statistical analysis plan. Sensitivity (SE), specificity (SP), positive predictive value (PPV) and negative predictive value (NPV) of the MSC were computed to evaluate the discriminatory performance of MSC to classify patients diagnosed with lung cancer versus disease-free subjects overall and for both LDCT and Observational arms of the study. For diagnostic performance, individuals categorized within the MSC-Low risk group were compared to those within either MSC-Intermediate or MSC-High. We also computed SE and SP for the combined use of binary MSC and LDCT, considering single positive and double positive tests.

In order to account for time dependency of MSC as a predictor of disease development, the SE, SP, PPV and NPV were calculated for the various time intervals from blood sample collection to lung cancer diagnosis (6, 12, 18 and 24 months), using the methodology described by Heagerty et al, Biometrics 56:337-344 (2000) and Zheng and Heagerty, Biometrics 63:332-341 (2007).

To determine the prognostic performance of MSC, all three risk groups were examined and a survival curve was obtained as the Kaplan-Meier estimator from the date of blood sample collection according to MSC among all 939 subjects. We estimated the heterogeneity of MSC in survival using Cox proportional hazards models, considering also models further adjusted for age and gender and the χ21 test between High/Intermediate and Low MSC was computed.

**D. Evaluation of hemolysis affecting plasma samples.** Plasma samples with the presence of hemolysis were removed from subsequent analyses because hemolysis of blood cells such as red blood cells (RBC) or platelets releases contaminating miRNAs (Kirschner et al., 2011; Pritchard et al., 2012). Two quality control (QC) measurements were used for this evaluation. First, in a pre-analytical step prior to RNA extraction, a spectrophotometric analysis was completed by measuring the absorbance at different wavelengths (414nm, 541nm, 576nm) to identify the presence and amount of free hemoglobin in the sample (Kirschner et al., 2011).

A second QC step was implemented to obtain even greater sensitivity of hemolysis by analyzing expression levels of hemolysis-related miRNAs contained within the miRNA signature classifier (MSC; mir-451, 486-5p, 16, 92a) for all samples. Plasma samples with expression levels of hemolysis-related miRNAs that exceeded 2 standard deviations from the overall mean of all samples were excluded from subsequent analyses. Samples with detectable spectrophotometrically measured hemolysis were also excluded in this second QC step. No difference in the frequency or in the amount of hemolysis as measured either spectrophotometrically or by hemolysis-related miRNAs analysis was observed in the cancer vs control samples.

In addition to the potential of contaminating miRNAs released by hemolysis, miRNA differentially expressed in plasma could simply reflect different blood cell counts (Pritchard, 2012). In order to analyze this hypothesis, miRNA ratios composed by neutrophil-expressed and RBC-expressed miRNAs (*Id*.) were compared with ratios between the levels of neutrophil and RBC obtained by complete blood count (CBC) from 23 lung cancer patients of this present study. As reported in Table 3, none of the miRNA ratios present in the signatures were found to have a significant correlation with respective CBC ratios.

**Table 3. Plasma miRNA ratios correlation to blood cell counts in 23 lung cancer patients. Pearson correlation coefficients of neutrophil vs. RBC miRNA ratios and neutrophil vs. RBC counts in the 23 samples are shown in the table. Statistically significant correlations (two tailed p-values < 0.05) are reported.**

| Ratios Neutrophil miRNA/RBC miRNA | Pearson correlation with cell counts Neutrophil/RBC |
|---|---|
| 197/16⁺ | 0.34 |
| 197/486-5p⁺ | 0.20 |
| 197/451⁺ | 0.15 |
| 197/92a⁺ | -0.07 |
| 142-3p/16 | 0.16 |
| 142-3p/486-5p | 0.01 |
| 142-3p/451 | 0.07 |
| 142-3p/92a | -0.10 |
| 140-5p/16 | 0.27 |
| 140-5p/486-5p | 0.15 |
| 140-5p/451 | 0.13 |
| 140-5p/92a | -0.11 |
| 17/16⁺ | 0.19 |
| 17/486-5p⁺ | 0.11 |
| 17/451⁺ | 0.10 |
| 17/92a⁺ | -0.19 |
| 21/16 | 0.43° |
| 21/486-5p | 0.14 |
| 21/451 | 0.23 |
| 21/92a | 0.06 |

| | |
|---|---|
| ⁺*Present in the signatures described herein* °*Two-tailed p-value for Pearson correlation < 0.05* | |

The second QC step involving analysis of hemolysis-related miRNAs may additionally or alternatively utilize a hemolysis miRNA signature comprising miRNAs contained within the MSC as described herein.

### i. Detection of hemolysis using an miRNA signature: Plasma collection

Samples of whole blood were collected, with addition of EDTA, and stored at room temperature for no longer than 1-2 hours before processing. Storage at reduced temperature is to be avoided because it may lead to non-specific release of miRNA. The samples were centrifuged at approximately 1250 times g at 4°C for 10 minutes to separate plasma, which was carefully transferred while avoiding material closest to the lymphocytic ring. The plasma was centrifuged again under the same conditions and then separated into aliquots, with avoidance of pelleted material, that were stored at -80 °C for up to 1 year or longer.

### ii. MiRNA expression levels in hemolyzed versus non-hemolyzed samples

To develop the miRNA signature for detection of hemolysis, 24 plasma samples were hemolyzed. Hemolysis was assessed by visual inspection. Hemolyzed samples were profiled for expression of a panel of miRNAs using custom made microfluidic cards. Expression of the miRNAs in the hemolyzed samples was compared to that in non-hemolyzed (based on visual assessment) samples from 98 disease-free individuals.

Four miRNAs (miR-16, miR-451, miR-486-5p, and miR-92a) were included in the development of the miRNA signature. Raw Ct values (a measure of expression level) are shown in Table 4. The 4 miRNAs, miR-16, miR-451, miR-486-5p and miR-92a, in addition to miR-140-3p, are significantly upregulated (p<0.001) in hemolyzed samples versus non-hemolyzed samples.

**Table 4. Raw Ct values for miRNAs in hemolyzed versus non-hemolyzed samples.**

| | NOTHAEMOLYSED | | HAEMOLYSED | | | |
|---|---|---|---|---|---|---|
| | Ct average | s.d. | Ct average | s.d. | p-value | Δ |
| miR-101 | 30.0 | 1.5 | 28.8 | 1.8 | 0.007 | 1.2 |
| miR-106a | 20.5 | 1.4 | 19.9 | 1.6 | 0.092 | 0.6 |
| miR-126 | 21.3 | 1.4 | 21.7 | 1.5 | 0.261 | -0.4 |
| miR-133a | 30.0 | 1.8 | 30.8 | 2.4 | 0.132 | -0.8 |
| miR-140-3p | 30.7 | 1.8 | 28.9 | 1.9 | < 0.001 | 1.8 |
| miR-140-5p | 25.8 | 1.3 | 25.8 | 1.6 | 0.862 | -0.1 |
| miR-142-3p | 21.5 | 1.5 | 22.3 | 1.8 | 0.030 | -0.9 |
| miR-145 | 26.1 | 1.6 | 26.6 | 1.8 | 0.260 | -0.5 |
| miR-148a | 28.9 | 1.5 | 28.6 | 1.5 | 0.486 | 0.2 |
| miR-15b | 24.9 | 1.5 | 25.0 | 1.6 | 0.823 | -0.1 |
| miR-16 | 20.7 | 1.5 | 18.3 | 2.2 | < 0.001 | 2.4 |
| miR-17 | 20.6 | 1.4 | 20.0 | 1.6 | 0.078 | 0.7 |
| miR-197 | 26.1 | 1.5 | 26.2 | 1.2 | 0.659 | -0.1 |
| miR-19b | 21.5 | 1.5 | 20.4 | 1.8 | 0.006 | 1.2 |
| miR-21 | 24.8 | 1.4 | 24.9 | 1.9 | 0.886 | -0.1 |
| miR-221 | 24.1 | 1.4 | 24.8 | 1.9 | 0.108 | -0.7 |
| miR-28-3p | 26.4 | 1.5 | 26.8 | 1.4 | 0.256 | -0.4 |
| miR-30b | 22.2 | 1.5 | 22.8 | 1.6 | 0.087 | -0.6 |
| miR-30c | 24.0 | 1.6 | 24.3 | 1.6 | 0.294 | -0.4 |
| miR-320 | 23.1 | 1.4 | 22.6 | 1.4 | 0.107 | 0.5 |
| miR-451 | 22.9 | 1.6 | 20.0 | 2.5 | < 0.001 | 2.9 |
| miR-486-5p | 23.5 | 1.6 | 20.6 | 2.2 | < 0.001 | 2.8 |
| miR-660 | 29.2 | 1.5 | 27.8 | 2.1 | 0.004 | 1.5 |
| miR-92a | 24.0 | 1.4 | 22.8 | 1.5 | < 0.001 | 1.3 |

### iii. Generation of miRNA ratios in the signature for hemolysis

In order to discriminate between hemolyzed versus non-hemolyzed samples using miRNA ratios, normal plasma samples containing serial dilutions of an *in vitro* hemolyzed plasma sample were analyzed for miRNA expression levels. Ratios of various miRNAs to each of the four miRNAs shown in Table 4 to be upregulated (miR-16, miR-451, miR-486-5p, and miR-92a) were calculated. MiR-126, miR-15b, miR-221, and miR-30b, were identified as the miRNAs whose ratios with the four upregulated miRNAs had the best correlation with hemolysis (Table 5).

**Table 5. Plasma miRNA expression level ratios representing hemolysis in vitro.**

| | Cut-off | 1/800 | 1/1600 | 1/3200 | 1/6400 | 1/12800 | 1/25600 | 1/51200 | 1/102400 | Not treated |
|---|---|---|---|---|---|---|---|---|---|---|
| 451/126 | 0.74 | 2.60 | 1.66 | 0.70 | 0.94 | 0.41 | 0.28 | -0.53 | -0.69 | -0.78 |
| 451/15b | 4.07 | 5.88 | 5.28 | 4.30 | 4.54 | 4.00 | 4.05 | 3.11 | 3.39 | 2.85 |
| 451/221 | 3.81 | 5.69 | 4.94 | 4.14 | 4.16 | 3.76 | 3.66 | 3.32 | 3.06 | 2.26 |
| 451/30b | 1.53 | 2.73 | 2.15 | 1.25 | 1.12 | 0.85 | 0.50 | -0.31 | -0.01 | -0.29 |
| 486-5p/126 | -0.34 | 2.25 | 1.47 | 0.41 | 0.33 | -0.11 | -0.02 | -0.27 | -0.75 | -1.00 |
| 486-5p/15b | 3.33 | 5.51 | 5.09 | 4.01 | 3.93 | 3.48 | 3.75 | 3.38 | 3.33 | 2.63 |
| 486-5p/221 | 2.63 | 5.32 | 4.74 | 3.85 | 3.55 | 3.23 | 3.36 | 3.59 | 2.99 | 2.04 |
| 486-5p/30b | 0.70 | 2.36 | 1.96 | 0.96 | 0.52 | 0.33 | 0.20 | -0.05 | -0.07 | -0.51 |
| 92a/126 | -1.86 | -0.54 | -1.09 | -1.84 | -1.84 | -1.80 | -1.93 | -2.47 | -2.77 | -2.70 |
| 92a/15b | 1.70 | 2.74 | 2.53 | 1.76 | 1.76 | 1.79 | 1.83 | 1.18 | 1.31 | 0.93 |
| 92a/221 | 1.08 | 2.55 | 2.18 | 1.60 | 1.38 | 1.55 | 1.45 | 1.39 | 0.97 | 0.34 |
| 92a/30b | -0.83 | -0.41 | -0.60 | -1.29 | -1.66 | -1.36 | -1.71 | -2.24 | -2.10 | -2.22 |
| 16/126 | 1.92 | 5.15 | 4.05 | 3.54 | 3.50 | 3.00 | 3.26 | 2.91 | 2.42 | 1.79 |
| 16/15b | 5.34 | 8.42 | 7.67 | 7.14 | 7.10 | 6.59 | 7.03 | 6.55 | 6.50 | 5.43 |
| 16/221 | 5.01 | 8.28 | 7.32 | 6.98 | 6.72 | 6.35 | 6.65 | 6.76 | 6.16 | 4.84 |
| 16/30b | 2.75 | 5.28 | 4.54 | 4.09 | 3.68 | 3.44 | 3.49 | 3.13 | 3.10 | 2.28 |
| | | | | | | | | | | |
| Ratios exceeding cut-off | | 16 | 16 | 13 | 13 | 10 | 9 | 8 | 6 | 1 |
| R414/375 | | 2.48 | 2.02 | 1.80 | 1.61 | 1.49 | 1.44 | 1.45 | 1.39 | 1.34 |

| | | | | | | | | | | |
|---|---|---|---|---|---|---|---|---|---|---|
| *Values are expressed as log₂(ratio)* | | | | | | | | | | |

Mean baseline miRNA ratios were calculated using the formula, mean ratio + 1.5 s.d. (standard deviation), from non-hemolyzed plasma samples from 98 disease-free individuals. These baseline ratios were set as the cut-off for each of the 16 miRNA ratios in developing the miRNA signature for hemolysis.

### iv. Using the hemolysis miRNA signature to detect hemolysis

The hemolysis miRNA signature can be used to detect hemolysis in plasma samples. Table 6 shows a list of 16 ratios composed of 8 miRNAs (miR-126, 15b, 30b, 221, 451, 16, 486-5p and 92a). In particular, the 4 miRNAs, miR-451, miR-486-5p, miR-16 and miR-92a, are all overexpressed in hemolyzed samples. This overexpression can be used as an indicator of hemolysis in a plasma sample. In addition, if 8, or more, out of 16 ratios shown in Table 6 exceed the cut-off value, then the plasma sample is positive for hemolysis.

**Table 6. miRNA signature for hemolysis**

| Ratios of miRNAs | Cut-off (log2) |
|---|---|
| 126/451 | < -0.74 |
| 15b/451 | < -4.07 |
| 221/451 | < -3.81 |
| 30b/451 | < -1.53 |
| 126/486 | < 0.34 |
| 15b/486 | < -3.33 |
| 221/486 | < -2.63 |
| 30b/486 | < -0.70 |
| 126/92a | < 1.86 |
| 15b/92a | < -1.70 |
| 221/92a | < -1.08 |
| 30b/92a | < 0.83 |
| 126/16 | < -1.92 |
| 15b/16 | < -5.34 |
| 221/16 | < -5.01 |
| 30b/16 | < -2.75 |

### v. Comparison of hemolysis miRNA signature versus hemoglobin absorbance methods for detecting hemolysis

Hemoglobin is known in the art to have an absorbance wavelength at 414 nm. One standard method used in both scientific and clinical practice to analyze hemolysis is the spectroscopic measurement at the wavelength of 414 nm (using an absorbance threshold of 0.2) of free hemoglobin in plasma samples. In this experiment, the absorbance at 414 nm was normalized against the absorbance at 375 nm in order to overcome the high background signal in some samples (e.g., in lipemic samples). A cut-off of 1.4 for the ratio of absorbance at 414 nm to absorbance at 375 nm was set.

As shown in the last row of Table 5, samples with a value of A414nm/A375nm greater than 1.40 corresponded to the miRNA signature wherein at least 8 out of 16 miRNA ratios exceeded their respective cut-offs. These settings were used to evaluate the power of the miRNA signature in distinguishing the 24 hemolyzed from 98 non-hemolyzed (based on visual inspection) plasma samples. The miRNA signature method exhibited a sensitivity of 0.88 and a specificity of 0.93. In regard to sensitivity, it was clear that the 24 samples that were visually assessed to be hemolyzed were indeed hemolyzed.

The hemolysis miRNA signature and spectrophotometry methods were compared using an independent series composed of a selection of 60 plasma samples that were visually hemolyzed (red, orange or dark yellow) and 43 plasma samples non-hemolyzed based on visual inspection. Concerning sensitivity (Table 7), the 5 most hemolyzed (red) samples were all recognized as positive for hemolysis by both methods. However, in evaluating the orange and dark yellow samples, the miRNA signature method was more sensitive (>90% versus 76% for the spectrophotometric method).

**Table 7. Comparing spectrophotometer and miRNAs results to evaluate hemolysis in 60 visually hemolyzed plasma samples.**

| | Red | Orange | Dark yellow |
|---|---|---|---|
| Total | 5 | 25 | 30 |
| R 414/375 | 5(100.0) | 19(76.0) | 23(76.7) |
| miRNAs | 5(100.0) | 23(92.0) | 29(96.7) |
| Concordance | 5(100.0) | 21(84.0) | 22(73.3) |

| | | | |
|---|---|---|---|
| *( ) percentages of the total that were detected by each method as being hemolyzed* | | | |

On the other hand, in the 43 plasma samples that were non-hemolyzed based on visual inspection (Table 8), results showed that 3 (7%) samples were positive for hemolysis using both methods, 1 (2%) sample was positive in only the spectrophotometric method, and 2 (5%) samples were positive in only the miRNA signature method. Forty (93%) samples had an agreement in hemolysis classification between the two methods. Excluding the 3 cases positive in both the methods, the spectrophotometric method had 97.5% specificity and the miRNA signature method had 95% specificity.

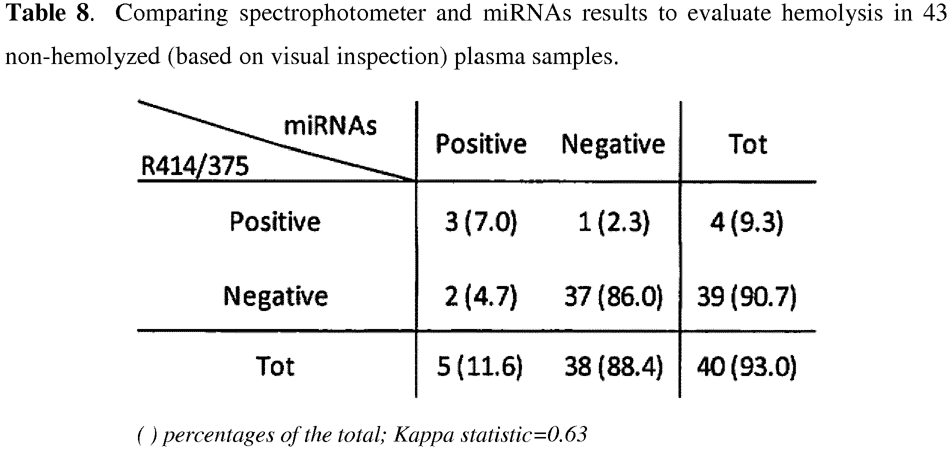

**E. MiRNA signatures classifier (MSC) algorithm.** The MSC algorithm is as described in detail herein. For the development of this algorithm, different gene expression ratios of 24 different miRNAs were generated starting from 4,950 ratios of 100 different miRNAs (stably) circulating in plasma in a training set of samples from lung cancer patients prospectively collected prior to or at diagnosis from the INT/IEO lung cancer screening trial as previously described (Boeri et al., 2011). The development of the pre- specified MSC algorithm used in this study was refined from that previous study in two ways.

First, samples with detectable hemolysis were removed from the original training set (Boeri et al., 2011). Subsequent to that publication, investigators reported that hemolysis affected accurate expression measurements of miRNAs in plasma and serum samples. Thus, to generate an optimal training set for MSC, 5 samples within the original training set were excluded due to detectable hemoglobin by spectrophotometric analysis and increase in hemolysis-related miRNA levels (as described herein). MiRNA ratios were then used in the optimal training set to develop the miRNA signatures for Risk of Disease (RD), Presence of Disease (PD), Risk of Aggressive Disease (RAD) and Presence of Aggressive Disease (PAD; Table 9).

**Table 9. Refined miRNA-ratios' signatures and corresponding cut-off values (log₂).**

| RD | Cut-off | RAD | Cut-off | PD | Cut-off | PAD | Cut-off |
|---|---|---|---|---|---|---|---|
| 197/660 | > 4.30 | 197/451 | > -1.75 | 106a/142-3p | > 2.02 | 197/486-5p | > -1.39 |
| 17/660 | > 9.26 | 28-3p/451 | > -2.41 | 106a/140-5p | > 5.50 | 197/451 | > -1.75 |
| 28-3p/660 | > 3.36 | 320/451 | > 1.00 | 106a/660 | > 9.32 | 197/660 | > 4.30 |
| 133a/660 | > 0.59 | 126/451 | > 2.85 | 106a/92a | > 3.97 | 17/486-5p | > 3.75 |
| 106a/660 | > 9.32 | 197/92a | > -1.28 | 142-3p/17 | < -1.95 | 17/451 | > 3.33 |
| 197/451 | > -1.88 | 28-3p/92a | > -1.81 | 140-5p/17 | < -5.58 | 17/660 | > 9.26 |
| 17/451 | > 3.33 | 320/92a | > 1.65 | 17/660 | > 9.26 | 106a/486-5p | > 3.75 |
| 28-3p/451 | > -2.49 | 126/92a | > 3.39 | 17/92a | > 3.81 | 106a/451 | > 3.33 |
| 133a/451 | > -5.49 | 142-3p/197 | < 3.25 | 142-3p/197 | < 3.25 | 106a/660 | > 9.32 |
| 19b/660 | > 8.38 | 142-3p/28-3p | < 3.75 | 140-5p/197 | < -0.34 | 126/486-5p | > 2.55 |
| 197/19b | > -4.12 | 126/142-3p | > 0.81 | 197/660 | > 4.30 | 126/451 | > 2.85 |
| 142-3p/15b | < 2.88 | 19b/451 | > 2.85 | 142-3p/28-3p | < 3.75 | 126/660 | > 8.55 |
| 15b/660 | > 5.00 | 197/660 | > 4.30 | 140-5p/28-3p | < 0.26 | 16/197 | < 5.00 |
| 320/660 | > 6.77 | 197/30c | > -1.41 | 28-3p/660 | > 3.36 | 140-5p/197 | < -0.34 |
| 126/660 | > 8.55 | 197/21 | > -0.40 | 126/142-3p | > 0.81 | 197/92a | > -1.28 |
| 140-3p/660 | > -0.21 | 17/451 | > 3.33 | 126/140-5p | > 4.76 | 197/30b | > -3.24 |
| 16/197 | < 5.00 | 106a/451 | > 3.33 | 126/660 | > 8.55 | 197/30c | > -1.41 |
| 197/92a | > -1.28 | 197/30b | > -3.24 | 142-3p/145 | < 3.62 | 19b/660 | > 8.38 |
| 17/92a | > 3.81 | 106a/142-3p | > 2.02 | 320/660 | > 6.77 | 28-3p/486-5p | > -2.40 |
| 133a/92a | > -4.18 | 142-3p/17 | < -1.95 | 142-3p/15b | < 2.88 | 28-3p/451 | > -2.41 |
| 101/140-3p | < -0.20 | 21/28-3p | < 0.70 | 19b/660 | > 8.38 | 16/17 | < -0.50 |
| 15b/30c | > -0.67 | 126/21 | > 3.98 | 142-3p/148a | < 6.21 | 106a/16 | > 0.55 |
| 106a/92a | > 3.97 | 197/19b | > -4.10 | 197/92a | > -1.28 | 19b/486-5p | > 2.85 |
| 15b/30b | > -2.47 | 28-3p/660 | > 3.36 | 142-3p/30b | < -0.40 | 19b/451 | > 2.85 |
| 15b/21 | > 0.36 | 21/221 | < -1.35 | 142-3p/21 | < 2.62 | 320/486-5p | > 1.40 |
| 106a/451 | > 3.33 | 145/197 | < -1.10 | 142-3p/221 | < 1.69 | 320/451 | > 1.00 |
| 15b/451 | > -0.90 | 28-3p/30c | > -1.99 | 133a/142-3p | > -7.20 | 320/660 | > 6.77 |
| | | 28-3p/30b | > -3.60 | | | 16/320 | < 1.71 |

| | | | | | | | |
|---|---|---|---|---|---|---|---|
| *RD: Risk of disease; RAD: risk of aggressive disease; PD: presence of disease; PAD: presence of aggressive disease.* *Ratio cut-off values were established for plasma samples stored at -80°C for at least 1 and up to 5 years.* | | | | | | | |

The second way the MSC algorithm development differed in this study from the previous study is that predefined ratio cut-off values were generated to obtain ≥80% specificity using plasma samples from a training set of 84 disease-free individuals from the MILD trial that were not included in the 939 subject validation cohort.

The use of plasma samples from single subjects, instead of pools (used in Boeri et al., 2011), allowed us to consider all of the 24 miRNAs originally identified in the training set and to generate accurate cut-offs. Therefore, the present study could include mir-101, 145 and 133a, which were excluded in the former validation set because of a high variability among the subjects within the control pools used in that study.

To build a three-level risk categorization for disease (MSC low, intermediate and high), the training set was also used to establish the minimum number of ratios exceeding the respective cut-off value needed to be considered positive: 10/27 for RD, 9/27 for PD, 14/28 for RAD, 14/28 for PAD. The three-level MSC was then defined as follows: Low risk (L) if RD^{neg} ∩ PD^{neg} ∩ RAD^{neg} ∩ PAD^{neg}; Intermediate risk (I) if RD^{pos} U PD^{pos} ∩ RAD^{neg} ∩ PAD^{neg}; or High risk (H) if RAD^{pos} U PAD^{pos}. These pre-specified risk groups were then used to test diagnostic and prognostic performance within an independent set of 939 subjects from the MILD screening trial.

**F. Characteristics of subjects.** Characteristics of 939 subjects with evaluable plasma samples enrolled in the MILD trial (2005-2012; LDCT: N=652; Observational: N=287), including 69 patients with lung cancer and 870 subjects without lung cancer, are indicated according to age, sex, and tobacco smoking status, duration and number of cigarettes/day (Table 10). Lung cancer patients were older than patients without lung cancer (p<0.0001), and the proportion of males was higher (81.2% vs. 63.3%; p=0.0029). Smoking status was not significantly different between with or without cancer groups, but subjects who developed cancer had smoked for a longer time (p<0.0001).

**Table 10. Distribution of 69 subjects with lung cancer and 870 subjects without lung cancer according to age, sex, and tobacco smoking. The Multicentric Italian Lung Detection (MILD) study, 2005-2012.**

| | Lung cancers | No lung cancer | p-value* |
|---|---|---|---|
| Age (years) | | | <0.0001 |
| <55 | 13 (18.8) | 377 (43.3) | |
| 55-59 | 15 (21.7) | 242 (27.8) | |
| 60-64 | 23 (33.3) | 165 (19.0) | |
| ≥65 | 18 (26.1) | 86 (9.9) | |
| *mean (sd)* | 60.9 (6.3) | 56.4 (5.8) | |
| *range* | 50-77 | 50-75 | |
| Sex | | | 0.0029 |
| female | 13 (18.8) | 319 (36.7) | |
| male | 56 (81.2) | 551 (63.3) | |
| Smoking status | | | 0.9 |
| ex-smoker | 14 (20.3) | 180 (20.7) | |
| current smoker | 55 (79.7) | 690 (79.3) | |
| Duration of smoking (years) | | | <0.0001 |
| <40 | 19 (27.5) | 500 (57.5) | |
| 40-49 | 41 (59.4) | 326 (37.5) | |
| ≥50 | 9 (13.1) | 44 (5.1) | |
| Cigarettes/day | | | 0.1201 |
| <20 | 14 (20.3) | 235 (27.0) | |
| 20-29 | 31 (44.9) | 440 (50.6) | |
| 30-39 | 11 (15.9) | 96 (11.0) | |
| ≥40 | 13 (18.8) | 99 (11.4) | |

| | | | |
|---|---|---|---|
| *( ) Percentage of subjects in each category on the total number of subjects with or without lung cancer.* **Estimated by χ² test* | | | |

For cancer patients, median time from randomization to diagnosis was 29 months (range 1-82), and median time from plasma sampling to diagnosis was 2 months (range 0-35). In lung cancer-free subjects, median time from randomization to plasma sampling was 44 months (range 0-58) and median time from plasma sampling to last follow-up was 27 months (range 3-41).

### Example 2: Diagnostic and prognostic performance of MSC

Evaluable plasma samples obtained prior to or at diagnosis from 939 subjects across LDCT and observational arms were analyzed using a real-time RT-PCR based assay with a pre-specified MSC algorithm of Low, Intermediate and High risk of cancer groups. MSC risk groups were examined for all 939 subjects according to lung cancer occurrence, lung cancer death, and tumor stage (Table 11). MSC Intermediate and High correctly classified 60 of 69 lung cancer patients with 87% SE, 81% SP, 27% PPV and 99% NPV. Of the 19 lung cancer patients that died during follow-up, 18 were positive at the MSC test, with 95% SE, 81% SP, 10% PPV and 100% NPV. No deaths due to causes other than lung cancer were observed during the follow-up. Comparative diagnostic performance of MSC for lung cancer detection within the two arms was similar with 88% SE, 80% SP, 31% PPV, 99% NPV and 82% SE, 83% SP, 16% PPV, 99% NPV for LDCT and Observational arms respectively.

**Table 11. Distribution of 69 subjects with lung cancer and 870 subjects without lung cancer according to lung cancer prevalence, lung cancer death, and lung cancer stage and miRNA signature classifier (MSC), with corresponding sensitivity (SE), specificity (SP), positive predictive value (PPV), and negative predictive value (NPV). The Multicentric Italian Lung Detection (MILD) study, 2005-2012.**

| | Total | MSC (risk of lung cancer) | | |
|---|---|---|---|---|
| | | High (H) | Intermediate (I) | Low (L) |
| All subjects | 939 | 63(6.7) | 159(16.9) | 717(76.4) |
| No lung cancer | 870 | 32(3.7) | 130(14.9) | 708(81.4) |
| Lung cancer | 69 | 31(44.9) | 29(42.0) | 9(13.0) |
| *performance** | | SE=87%, SP=81%, PPV=27%, NPV=99% | | |
| Lung cancer deaths⁺ | 19 | 12(63.2) | 6(31.6) | 1(5.3)° |
| Lung cancer, stage I^{≠} | 37 | 14(37.8) | 19(51.4) | 4(10.8) |
| Lung cancer, stage II-III^{≠} | 12 | 5(41.7) | 4(33.3) | 3(25.0) |
| Lung cancer, stage IV^{≠} | 19 | 11(57.9) | 6(31.6) | 2(10.5) |

| | | | | |
|---|---|---|---|---|
| *() Percentages of the subjects with MSC risk groups (L, I, & H) within the total number of subjects for each category.* **SE, SP, PPV and NPV were calculated combining pre-specified MSC High and Intermediate versus Low risk.* ⁺*P*=*0.0366, based on the Cochran-Armitage test for trend in the proportion of deaths across strata of MSC risk groups among subjects with lung cancer.* *° plasma sample obtained 30 months before disease detection.* ^{≠} *tumor stage information was not available in one patient. p*=*0.49 for association of MSC with tumor stage* | | | | |

Across all three MSC risk groups, a significant trend in the proportion of death-by-disease was observed with an increasing proportion of lung cancer deaths associated with Low, Intermediate and High respectively (p=0.0336). MSC risk groups were not significantly associated (p=0.40) with varying tumor stage (I, II-III or IV; Table 11).

No significant differences were observed between MSC risk groups and histological subtypes (χ² =1.60, p=0.4485), and between adenocarcinoma and squamous cell carcinoma (χ² = 0.55, p=0.759). Time dependency analysis of diagnostic performance of MSC, showed similar values of SE, SP, PPV and NPV at 6, 12, 18 and 24 month intervals between blood sampling and lung cancer diagnosis (Table 12).

**Table 12. Time dependency analysis of miRNA signature classifier (MSC), with sensitivity (SE), specificity (SP), positive predictive value (PPV), and negative predictive value (NPV) calculated at 6, 12, 18 and 24 months from blood sampling to lung cancer detection.**

| Months from blood sampling to lung cancer detection | SE | SP | PPV | NPV |
|---|---|---|---|---|
| 6 | 83% | 80% | 18% | 99% |
| 12 | 86% | 81% | 22% | 99% |
| 18 | 86% | 81% | 23% | 99% |
| 24 | 87% | 81% | 25% | 99% |

### Example 3: Complementary diagnostic performance of LDCT and MSC

Restricting the analysis to the total of 652 subjects in the LDCT arm, LDCT identified 46 of 58 lung cancer subjects missing 3 patients within the 251 subjects with no pulmonary nodule detected and 9 patients because of an interval cancer for a SE of 79% (Table 13). The three cancers with "no pulmonary nodule" comprised of one non-solid lesion, one mediastinal adenopathy, and one pleural effusion. Pre-specified binary risk groups of MSC (considering High and Intermediate versus Low) identified 40 of 46 LDCT-detected cancers, 8 of 9 interval cancers and all 3 subjects with "no pulmonary nodule".

**Table 13. Distribution of 939 subjects according to miRNA signature classifier (MSC) and low-dose computed tomography (LDCT), by LDCT (including screen-detected and non-screen detected lung cancers) and Observational arms. The Multicentric Italian Lung Detection (MILD) study, 2005-2012.**

| | TOTAL | No lung cancer | | | | Lung cancer | | | |
|---|---|---|---|---|---|---|---|---|---|
| | | Overall | | MSC | | Overall | | MSC | |
| | | | High | Intermediate | Low | | High | Intermediate | Low |
| **LDCT arm** | 652 | 594 | 22 | 94 | 478 | 58 | 27 | 24 | 7 |
| LDCT-detected | 643 | 594 | 22 | 94 | 478 | 49 | 22 | 21 | 6 |
| No nodule | 251 | 248 | 7 | 42 | 199 | 3 | 2 | 1 | 0 |
| Nodule diameter (mm) | | | | | | | | | |
| ≤ 5 | 231 | 231 | 12 | 33 | 186 | 0 | 0 | 0 | 0 |
| > 5 - ≤ 10 | 102 | 94 | 2 | 16 | 76 | 8 | 4 | 2 | 2 |
| > 10 | 59 | 21 | 1 | 3 | 17 | 38 | 16 | 18 | 4 |
| Interval cancer | 9 | | | | | 9 | 5 | 3 | 1 |
| **Observational arm** | 287 | 276 | 10 | 36 | 230 | 11 | 4 | 5 | 2 |
| | | | | | | | | | |
| TOTAL | 939 | 870 | 32 | 130 | 708 | 69 | 31 | 29 | 9 |

LDCT had a SP of 81% for the clinically actionable subgroup of non-calcified nodules >5 mm and an associated false positive rate of 19.4% (115/594) (Table 13). When double-positive (LDCT and MSC) subjects were considered, the false positive rate decreased to 3.7% (22/594), with a decrease in SE (40/58, 69%). Conversely, considering as positive a subject with at least one positive test (LDCT or MSC), the combined use of LDCT and MSC identified 57 of 58 cases, with a SE of 98% and a SP of 65%. On the other hand, MSC detected 9 of 11 (82%) lung cancers that occurred in the observational arm (Table 13).

### Example 4: Association of MSC risk groups with survival

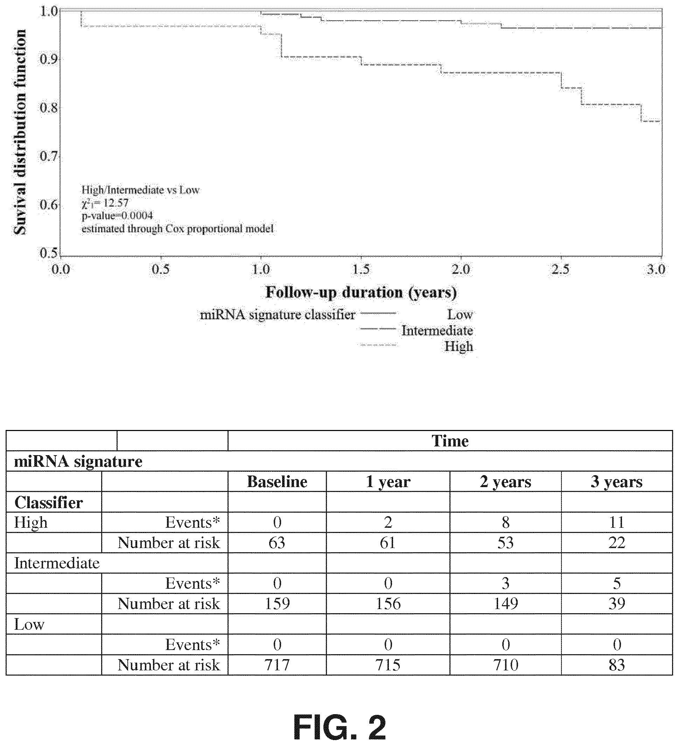

Analysis was completed to determine the prognostic performance of the three pre-defined MSC risk groups to predict overall survival from plasma samples collected for all subjects with three year follow-up (N=939). Two-year survival was 100% for subjects with Low MSC, 98% for Intermediate MSC and 87% for High MSC, while three-year survival was 100%, 97% and 77% for Low, Intermediate and High respectively (FIG. 2). The difference in survival between High/Intermediate and Low MSC was statistically significant (χ² =49.53, P<0.0001). The heterogeneity was still significant after adjustment for age and gender (χ²=12.57, P=0.0004).

### Example 5: Analysis

The diagnostic characteristic of high sensitivity coupled with a NPV of 99% indicates that MSC is a clinically validated screening test. Moreover, the diagnostic performance of MSC as a predictor of lung cancer development was confirmed by the time dependency analysis.

MSC identified subjects with a high likelihood of death-by-disease. As a binary diagnostic, MSC had a SE of 95% and NPV of 100% for death-by-disease for 939 subjects across both arms. Furthermore, the MSC risk groups were associated with significantly different survival at 3 years for the entire cohort of 939 subjects (100%, 97% and 77% for Low, Intermediate and High MSC respectively). These findings demonstrate that plasma miRNAs identify both malignancy and aggressiveness of the tumor.

Three small European randomized trials, including MILD, have reported to-date non-significant mortality reductions (Infante et al., 2009; Saghir et al., 2012; Pastorino et al., 2012). The National Lung Screening Trial (NSLT), a randomized clinical screening trial enrolling 53,454 persons with 3 rounds of LDCT annual screening of versus chest radiographs, demonstrated a 20% reduction of lung cancer mortality (Aberle et al., 2011). After 3 rounds of screening, 24.2% of subjects were classified as positive with 96.4% of these being a false positive with the need to screen 320 subjects to prevent 1 lung cancer death.

In a systematic review of all randomized clinical trials that examined the benefits and harms of LDCT screening, the average nodule detection rate was 20%, with 90% of nodules being benign (Bach et al., JAMA 307:2418-2429, 2012). In this study, MSC classified 74% (485) as Low risk among the 652 individuals in the LDCT arm: 478 were true negative and 7 were false negative. MSC was able to identify 8 out of 9 interval cancers undetected by LDCT. Thus, integration of MSC and LDCT (at least one test positive) would raise screening sensitivity from 87% for MSC and 84% for LDCT alone, to 98% (57/58), with a false positive rate of 35%. Conversely, considering double positive subjects (MSC and LDCT positive), the integration of MSC with LDCT would reduce the false positive rate of LDCT screening over 5-fold. Specifically, the frequency of double false-positive MSC and LDCT was only 3.7% (22/594) as compared with 19.7% (115/594) of LDCT alone, reducing however SE to 69%.

Consequently, MSC could complement LDCT screening by reducing false-positive results and enable greater standardization of diagnostic algorithms, thereby decreasing health care costs. Moreover, further repetitions of MSC rather than LDCT could be proposed for individuals with a Low MSC, given the absence of mortality at three years for Low MSC subjects. The present blinded study of a pre-specified plasma-based assay represents the largest study testing a biomarker within a LDCT screening trial, and demonstrates the superior diagnostic performance of LDCT if combined with MSC.

## Claims

1. A method of determining the presence of a pulmonary tumor in a subject, the method comprising:
(a) determine the expression ratio of an miRNA pair in a biological sample from the subject;
(b) compare the expression ratio from step (a) with a cut-off value determined from the average ratio of a plurality of corresponding miRNA pairs from a plurality of control samples;
(c) assign a positive score for the expression ratio of the miRNA pair in step (a) if the ratio exceeds the cut-off value in step (b), or assign a non-positive score for the expression ratio of the miRNA pair in step (a) if the ratio does not exceed the cut-off value in step (b);
(d) repeat steps (a) through (c) for additional miRNA pairs until either (1) at least nine miRNA pair expression ratios are assigned a positive score, or (2) after the comparison of the expression ratios of 27 miRNA pairs less than nine miRNA pair expression ratios are assigned a positive score,
wherein the miRNA pairs comprise one or more or each of 106a/140-5p, 106a/142-3p, 126/140-5p, 126/142-3p, 133a/142-3p, 140-5p/17, 142-3p/148a, 142-3p/15b, 142-3p/17, 142-3p/21, 142-3p/221, 142-3p/30b, and 320/660, or the inverse ratios thereof; and
(e) categorize the presence of a pulmonary tumor as (i) positive if at least nine of the miRNA pair expression ratios are assigned a positive score, or (ii) negative if less than nine miRNA pair expression ratios are assigned a positive score.

2. The method of claim 1, wherein the miRNA pairs further comprise one or more or each of 106a/660, 106a/92a, 126/660, 140-5p/197, 140-5p/28-3p, 142-3p/145, 142-3p/197, 142-3p/28-3p, 17/660, 17/92a, 197/660, 197/92a, 19b/660, and 28-3p/660, or the inverse ratios thereof.

3. A method of determining the presence of an aggressive pulmonary tumor in a subject comprising:
(a) determine the expression ratio of an miRNA pair in a biological sample from the subject;
(b) compare the expression ratio from step (a) with a cut-off value determined from the average ratio of a plurality of corresponding miRNA pairs from a plurality of control samples;
(c) assign a positive score for the expression ratio of the miRNA pair in step (a) if the ratio exceeds the cut-off value in step (b), or assign a non-positive score for the expression ratio of the miRNA pair in step (a) if the ratio does not exceed the cut-off value in step (b);
(d) repeat steps (a) through (c) for additional miRNA pairs until either (1) at least fourteen miRNA pair expression ratios are assigned a positive score, or (2) after the comparison of the expression ratios of 28 miRNA pairs less than fourteen miRNA pair expression ratios are assigned a positive score,
wherein the miRNA pairs comprise one or more or each of 106a/16, 106/660, 16/17, 16/320, 17/660, 197/30b, 197/30c, 320/451, 320/486-5p, and 320/660, or the inverse ratios thereof; and
(e) categorize the presence of an aggressive pulmonary tumor as (i) positive if at least fourteen of the miRNA pair expression ratios are assigned a positive score, or (ii) negative if less than fourteen miRNA pair expression ratios are assigned a positive score.

4. The method of claim 3, wherein the miRNA pairs further comprise one or more or each of 106a/451, 106a/486-5p, 126/451, 126/486-5p, 126/660, 140-5p/197, 16/197, 17/451, 17/486-5p, 197/451, 197/486-5p, 197/660, 197/92a, 19b/451, 19b/486-5p, 19b/660, 28-3p/451, and 28-3p/486-5p, or the inverse ratios thereof.

5. A method of determining the risk of manifesting a pulmonary tumor in a subject, the method comprising:
(a) determine the expression ratio of an miRNA pair in a biological sample from the subject;
(b) compare the expression ratio from step (a) with a cut-off value determined from the average ratio of a plurality of corresponding miRNA pairs from a plurality of control samples;
(c) assign a positive score for the expression ratio of the miRNA pair in step (a) if the ratio exceeds the cut-off value in step (b), or assign a non-positive score for the expression ratio of the miRNA pair in step (a) if the ratio does not exceed the cut-off value in step (b);
(d) repeat steps (a) through (c) for additional miRNA pairs until either (1) at least ten miRNA pair expression ratios are assigned a positive score, or (2) after the comparison of the expression ratios of 27 miRNA pairs, less than ten miRNA pair expression ratios are assigned a positive score,
wherein the miRNA pairs comprise one or more or each of 133a/92a, 15b/21, 15b/30b, 15b/30c, 16/197, and 28-3p/451, or the inverse ratios thereof; and
(e) categorize the risk of manifesting a pulmonary tumor as (i) positive if at least ten miRNA pair expression ratios are assigned a positive score, or (ii) negative if less than ten miRNA expression ratios are assigned a positive score.

6. The method of claim 5, wherein the miRNA pairs further comprise one or more or each of 101/140-3p, 106a/451, 106a/660, 106a/92a, 126/660, 133a/451, 133a/660, 140-3p/660, 142-3p/15b, 15b/451, 15b/660, 17/451, 17/660, 17/92a, 197/19b, 197/451, 197/660, 197/92a, 19b/660, 28-3p/660, and 320/660, or the inverse ratios thereof.

7. A method of determining the risk of manifesting an aggressive pulmonary tumor in a subject, the method comprising:
(a) determine the expression ratio of an miRNA pair in a biological sample from the subject;
(b) compare the expression ratio from step (a) with a cut-off value determined from the average ratio of a plurality of corresponding miRNA pairs from a plurality of control samples;
(c) assign a positive score for the expression ratio of the miRNA pair in step (a) if the ratio exceeds the cut-off value in step (b), or assign a non-positive score for the expression ratio of the miRNA pair in step (a) if the ratio does not exceed the cut-off value in step (b);
(d) repeat steps (a) through (c) for additional miRNA pairs until either (1) at least fourteen miRNA pair expression ratios are assigned a positive score, or (2) after the comparison of the expression ratios of 28 miRNA pairs less than fourteen miRNA pair expression ratios are assigned a positive score,
wherein the miRNA pairs comprise one or more or each of 106a/142-3p, 126/142-3p, 126/21, 126/92a, 142-3p/17, 142-3p/197, 142-3p/28-3p, 197/19b, 197/660, and 28-3p/660, or the inverse ratios thereof; and
(e) categorize the risk of manifesting an aggressive pulmonary tumor as (i) positive if at least fourteen miRNA pair expression ratios are assigned a positive score, or (ii) negative if less than fourteen miRNA pair expression ratios are assigned a positive score.

8. The method of claim 7, wherein the miRNA pairs further comprise one or more or each of 106a/451, 126/451, 145/197, 17/451, 197/21, 197/30b, 197/30c, 197/451, 197/92a, 19b/451, 21/221, 21/28-3p, 28-3p/30b, 28-3p/30c, 28-3p/451, 28-3p/92a, 320/451, and 320/92a, or the inverse ratios thereof.

9. A method for predicting the risk of developing or having a pulmonary tumor in a subject comprising:
(a) determining the presence of a pulmonary tumor signature of claim 1;
(b) determining the presence of an aggressive pulmonary tumor signature of claim 3;
(c) determining the risk of manifesting a pulmonary tumor signature of claim 5;
(d) determining the risk of manifesting an aggressive pulmonary tumor signature of claim 7;
(e) categorizing the subject as low risk of developing or having a pulmonary tumor if none of the signatures of steps (a)-(d) are categorized as positive;
(f) categorizing the subject as having an intermediate risk of developing or having a pulmonary tumor if at least the presence of a pulmonary tumor signature of step (a) is positive, or the risk of manifesting a pulmonary tumor signature of step (c) is positive; and both the presence of an aggressive pulmonary tumor signature of step (b) and the risk of manifesting an aggressive pulmonary tumor signature of step (d) are negative; and
(g) categorizing the subject as high risk of developing or having a pulmonary tumor if at least the presence of an aggressive pulmonary tumor signature of step (b) is positive or the risk of manifesting an aggressive pulmonary tumor signature of step (d) is positive.

10. The method of any one of claims 1, 3, 5 or 7, wherein the cut-off value is determined as the mean ratio from the plurality of control samples, the mean ratio from the plurality of control samples +/- one standard deviation or the median ratio from a plurality of control samples.

11. The method of claim 1, 3, 5 or 7, wherein the miRNA pair expression ratio is determined by making cDNA copies of each miRNA in each miRNA pair using reverse transcriptase-polymerase chain reaction (RT-PCR), preferably using real time PCR, more preferably using TaqMan probes.

12. The method of any one of claims 1, 3, 5 or 7, wherein the biological sample is a biological fluid preferably selected from urine, blood, plasma or serum, more preferably plasma or serum.

13. The method of claim 12, wherein when the biological sample is plasma or serum hemolysis is determined in the biological sample and the sample is not subjected to the method if detectable hemolysis is present.

14. The method of claim 13, wherein hemolysis is determined spectrophotometrically or by analyzing expression levels of hemolysis-related miRNAs that are upregulated in hemolyzed samples or both.

15. The method of claim 14, wherein the hemolysis-related miRNAs comprise a plurality of the miRNAs miR-451, miR-486-5p, miR-16, miR-92a or miR-140-3p or wherein the hemolysis-related miRNAs are miR-451, miR-486-5p, miR-16, and miR-92a.

16. The method of claim 14, further comprising determining expression levels of a plurality of normalizing miRNAs that are not upregulated in hemolyzed samples, to normalize the expression levels of the hemolysis-related miRNAs, optionally wherein the normalizing miRNAs comprise miR-126, miR-15b, miR-221 and miR-30b, optionally wherein hemolysis is further determined by
(a) determining the expression ratio of each of 16 miRNA pairs consisting of each of miR-451, miR-486-5p, miR-16, and miR-92a paired with each of miR-126, miR-15b, miR-221 and miR-30b in the sample;
(b) comparing each of the 16 expression ratios from step (a) with a cut-off value determined for each expression ratio from the average ratio of a plurality of corresponding miRNA pairs from a plurality of control samples;
(c) for each of the 16 miRNA pairs, assign a positive score for the expression ratio in step (a) if the ratio exceeds the cut-off value in step (b), or assign a non-positive score for the expression ratio in step (a) if the ratio does not exceed the cut-off value in step (b); and
(d) categorizing the sample as (i) having hemolysis if eight or more out of the 16 ratios exceed the cutoff value, or (ii) not having hemolysis if fewer than eight out of the 16 ratios exceed the cutoff value.

17. The method of any one of claims 1, 3, 5 or 7, further comprising screening the subject for lung cancer using a system that does not comprise analysis of miRNA expression, prior to, or concurrently with miRNA expression analysis, optionally wherein the system comprises a blood test, an x-ray, computed tomography, positron emission tomography, thoracentesis, bronchoscopy, fine-needle aspiration, thoracoscopy, thoracotomy, mediastinoscopy, preferably low-dose computed tomography (LDCT).

18. The method of any one of claims 1, 3, 5 or 7, wherein the method is performed on at least two different biological samples from the subject, preferably wherein at least one of the at least two different biological samples is taken from the subject after the subject has been treated for lung cancer.

19. The method of any one of claims 1, 3, 5, 7 or 9, wherein the subject is not treated for lung cancer if a negative presence or risk is determined, or if a low risk is determined; or the subject is treated for lung cancer if a positive presence or risk is determined, or if an intermediate or high risk is determined.

## Patentansprüche

1. Verfahren zum Bestimmen des Vorhandenseins eines Lungentumors bei einem Subjekt, wobei das Verfahren umfasst:
(a) Bestimmen des Expressionsverhältnisses eines miRNA-Paares in einer biologischen Probe von dem Subjekt;
(b) Vergleichen des Expressionsverhältnisses aus Schritt (a) mit einem Schwellenwert, der aus dem durchschnittlichen Verhältnis einer Vielzahl von entsprechenden miRNA-Paaren aus einer Vielzahl von Kontrollproben bestimmt wird;
(c) Zuweisen eines positiven Scores für das Expressionsverhältnis des miRNA-Paares in Schritt (a), wenn das Verhältnis den Schwellenwert in Schritt (b) übersteigt, oder Zuweisen eines nicht positiven Scores für das Expressionsverhältnis des miRNA-Paares in Schritt (a), wenn das Verhältnis den Schwellenwert in Schritt (b) nicht übersteigt;
(d) Wiederholen der Schritte (a) bis (c) für zusätzliche miRNA-Paare, bis entweder (1) mindestens neun miRNA-Paar-Expressionsverhältnissen ein positiver Score zugewiesen wird, oder (2) nach dem Vergleich der Expressionsverhältnisse von 27 miRNA-Paaren weniger als neun miRNA-Paar-Expressionsverhältnissen ein positiver Score zugewiesen wird,
wobei die miRNA-Paare eines oder mehrere oder jedes von 106a/140-5p, 106a/142-3p, 126/140-5p, 126/142-3p, 133a/142-3p, 140-5p/17, 142-3p/148a, 142-3p/15b, 142-3p/17, 142-3p/21, 142-3p/221, 142-3p/30b und 320/660 oder die umgekehrten Verhältnisse davon umfassen; und
(e) Kategorisieren des Vorhandenseins eines Lungentumors als (i) positiv, wenn mindestens neun der miRNA-Paar-Expressionsverhältnisse ein positiver Score zugewiesen wird, oder (ii) negativ, wenn weniger als neun miRNA-Paar-Expressionsverhältnissen ein positiver Score zugewiesen wird.

2. Verfahren nach Anspruch 1, wobei die miRNA-Paare ferner eines oder mehrere oder jedes von 106a/660, 106a/92a, 126/660, 140-5p/197, 140-5p/28-3p, 142-3p/145, 142-3p/197, 142-3p/28-3p, 17/660, 17/92a, 197/660, 197/92a, 19b/660 und 28-3p/660 oder die umgekehrten Verhältnisse davon umfassen.

3. Verfahren zum Bestimmen des Vorhandenseins eines aggressiven Lungentumors bei einem Subjekt, wobei das Verfahren umfasst:
(a) Bestimmen des Expressionsverhältnisses eines miRNA-Paares in einer biologischen Probe von dem Subjekt;
(b) Vergleichen des Expressionsverhältnisses aus Schritt (a) mit einem Schwellenwert, der aus dem durchschnittlichen Verhältnis einer Vielzahl von entsprechenden miRNA-Paaren aus einer Vielzahl von Kontrollproben bestimmt wird;
(c) Zuweisen eines positiven Scores für das Expressionsverhältnis des miRNA-Paares in Schritt (a), wenn das Verhältnis den Schwellenwert in Schritt (b) übersteigt, oder Zuweisen eines nicht positiven Scores für das Expressionsverhältnis des miRNA-Paares in Schritt (a), wenn das Verhältnis den Schwellenwert in Schritt (b) nicht übersteigt;
(d) Wiederholen der Schritte (a) bis (c) für zusätzliche miRNA-Paare, bis entweder (1) mindestens vierzehn miRNA-Paar-Expressionsverhältnissen ein positiver Score zugewiesen wird, oder (2) nach dem Vergleich der Expressionsverhältnisse von 28 miRNA-Paaren weniger als vierzehn miRNA-Paar-Expressionsverhältnissen ein positiver Score zugeordnet wird,
wobei die miRNA-Paare eines oder mehrere oder jedes von 106a/16, 106/660, 16/17, 16/320, 17/660, 197/30b, 197/30c, 320/451, 320/486-5p und 320/660 oder die umgekehrten Verhältnisse davon umfassen; und
(e) Kategorisieren des Vorhandenseins eines aggressiven Lungentumors als (i) positiv, wenn mindestens vierzehn der miRNA-Paar-Expressionsverhältnisse ein positiver Score zugewiesen wird, oder (ii) negativ, wenn weniger als vierzehn miRNA-Paar-Expressionsverhältnissen ein positiver Score zugewiesen wird.

4. Verfahren nach Anspruch 3, wobei die miRNA-Paare ferner eines oder mehrere oder jedes von 106a/451, 106a/486-5p, 126/451, 126/486-5p, 126/660, 140-5p/197, 16/197, 17/451, 17/486-5p, 197/451, 197/486-5p, 197/660, 197/92a, 19b/451, 19b/486-5p, 19b/660, 28-3p/451 und 28-3p/486-5p oder die umgekehrten Verhältnisse davon umfassen.

5. Verfahren zum Bestimmen des Risikos der Manifestation eines Lungentumors bei einem Subjekt, wobei das Verfahren umfasst:
(a) Bestimmen des Expressionsverhältnisses eines miRNA-Paares in einer biologischen Probe von dem Subjekt;
(b) Vergleichen des Expressionsverhältnisses aus Schritt (a) mit einem Schwellenwert, der aus dem durchschnittlichen Verhältnis einer Vielzahl von entsprechenden miRNA-Paaren aus einer Vielzahl von Kontrollproben bestimmt wird;
(c) Zuweisen eines positiven Scores für das Expressionsverhältnis des miRNA-Paares in Schritt (a), wenn das Verhältnis den Schwellenwert in Schritt (b) übersteigt, oder Zuweisen eines nicht positiven Scores für das Expressionsverhältnis des miRNA-Paares in Schritt (a), wenn das Verhältnis den Schwellenwert in Schritt (b) nicht übersteigt;
(d) Wiederholen der Schritte (a) bis (c) für zusätzliche miRNA-Paare bis entweder (1) mindestens zehn miRNA-Paar-Expressionsverhältnissen ein positiver Score zugewiesen wird, oder (2) nach dem Vergleich der Expressionsverhältnisse von 27 miRNA-Paaren weniger als zehn miRNA-Paar-Expressionsverhältnissen ein positiver Score zugewiesen wird,
wobei die miRNA-Paare eines oder mehrere oder jedes von 133a/92a, 15b/21, 15b/30b, 15b/30c, 16/197 und 28-3p/451 oder die umgekehrten Verhältnisse davon umfassen; und
(e) Kategorisieren des Risikos der Manifestation eines Lungentumors als (i) positiv, wenn mindestens zehn miRNA-Paar-Expressionsverhältnissen ein positiver Score zugewiesen wird, oder (ii) negativ, wenn weniger als zehn miRNA-Paar-Expressionsverhältnissen ein positiver Score zugewiesen wird.

6. Verfahren nach Anspruch 5, wobei die miRNA-Paare ferner eines oder mehrere oder jedes von 101/140-3p, 106a/451, 106a/660, 106a/92a, 126/660, 133a/451, 133a/660, 140-3p/660, 142-3p/15b, 15b/451, 15b/660, 17/451, 17/660, 17/92a, 197/19b, 197/451, 197/660, 197/92a, 19b/660, 28-3p/660 und 320/660 oder die umgekehrten Verhältnisse davon umfassen.

7. Verfahren zum Bestimmen des Risikos der Manifestation eines aggressiven Lungentumors bei einem Subjekt, wobei das Verfahren umfasst:
(a) Bestimmen des Expressionsverhältnisses eines miRNA-Paares in einer biologischen Probe von dem Subjekt;
(b) Vergleichen des Expressionsverhältnisses aus Schritt (a) mit einem Schwellenwert, der aus dem durchschnittlichen Verhältnis einer Vielzahl von entsprechenden miRNA-Paaren aus einer Vielzahl von Kontrollproben bestimmt wird;
(c) Zuweisen eines positiven Scores für das Expressionsverhältnis des miRNA-Paares in Schritt (a), wenn das Verhältnis den Schwellenwert in Schritt (b) übersteigt, oder Zuweisen eines nicht positiven Scores für das Expressionsverhältnis des miRNA-Paares in Schritt (a), wenn das Verhältnis den Schwellenwert in Schritt (b) nicht übersteigt;
(d) Wiederholen der Schritte (a) bis (c) für zusätzliche miRNA-Paare bis entweder (1) mindestens vierzehn miRNA-Paar-Expressionsverhältnissen ein positiver Score zugewiesen wird, oder (2) nach dem Vergleich der Expressionsverhältnisse von 28 miRNA-Paaren weniger als vierzehn miRNA-Paar-Expressionsverhältnissen ein positiver Score zugeordnet wird,
wobei die miRNA-Paare eines oder mehrere oder jedes von 106a/142-3p, 126/142-3p, 126/21, 126/92a, 142-3p/17, 142-3p/197, 142-3p/28-3p, 197/19b, 197/660 und 28-3p/660 oder die umgekehrten Verhältnisse davon umfassen; und
(e) Kategorisieren des Risikos der Manifestation eines aggressiven Lungentumors als (i) positiv, wenn mindestens vierzehn der miRNA-Paar-Expressionsverhältnisse ein positiver Score zugewiesen wird, oder (ii) negativ, wenn weniger als vierzehn miRNA-Paar-Expressionsverhältnissen ein positiver Score zugewiesen wird.

8. Verfahren nach Anspruch 7, wobei die miRNA-Paare ferner eines oder mehrere oder jedes von 106a/451, 126/451, 145/197, 17/451, 197/21, 197/30b, 197/30c, 197/451, 197/92a, 19b/451, 21/221, 21/28-3p, 28-3p/30b, 28-3p/30c, 28-3p/451, 28-3p/92a, 320/451 und 320/92a oder die umgekehrten Verhältnisse davon umfassen.

9. Verfahren zum Vorhersagen des Risikos der Entwicklung oder des Aufweisens eines Lungentumors bei einem Subjekt, wobei das Verfahren umfasst:
(a) Bestimmen des Vorhandenseins einer Lungentumorsignatur nach Anspruch 1;
(b) Bestimmen des Vorhandenseins einer aggressiven Lungentumorsignatur nach Anspruch 3;
(c) Bestimmen des Risikos der Manifestation einer Lungentumorsignatur nach Anspruch 5;
(d) Bestimmen des Risikos der Manifestation einer aggressiven Lungentumorsignatur nach Anspruch 7;
(e) Kategorisieren des Subjekts als geringes Risiko der Entwicklung oder des Aufweisens eines Lungentumors, wenn keine der Signaturen der Schritte (a)-(d) als positiv kategorisiert werden;
(f) Kategorisieren des Subjekts als ein intermediäres Risiko aufweisend, einen Lungentumor zu entwickeln oder aufzuweisen, wenn mindestens das Vorhandensein einer Lungentumorsignatur von Schritt (a) positiv ist, oder das Risiko der Manifestation einer Lungentumorsignatur von Schritt (c) positiv ist; und sowohl das Vorhandensein einer aggressiven Lungentumorsignatur von Schritt (b) und das Risiko der Manifestation einer aggressiven Lungentumorsignatur von Schritt (d) negativ sind; und
(g) Kategorisieren des Subjekts als ein hohes Risiko aufweisend, einen Lungentumor zu entwickeln oder aufzuweisen, wenn mindestens das Vorhandensein einer aggressiven Lungentumorsignatur von Schritt (b) positiv ist oder das Risiko der Manifestation einer aggressiven Lungentumorsignatur von Schritt (d) positiv ist.

10. Verfahren nach einem der Ansprüche 1, 3, 5 oder 7, wobei der Schwellenwert als das mittlere Verhältnis aus der Vielzahl von Kontrollproben, das mittlere Verhältnis aus der Vielzahl von Kontrollproben +/- einer Standardabweichung oder das mediane Verhältnis aus einer Vielzahl von Kontrollproben bestimmt wird.

11. Verfahren nach Anspruch 1, 3, 5 oder 7, wobei das miRNA-Paar-Expressionsverhältnis bestimmt wird durch die Herstellung von cDNA-Kopien von jeder miRNA in jedem miRNA-Paar unter Verwendung einer Reverse Transkriptase-Polymerase-Kettenreaktion (RT-PCR), bevorzugt unter Verwendung von Echtzeit-PCR, bevorzugter unter Verwendung von TaqMan-Sonden.

12. Verfahren nach einem der Ansprüche 1, 3, 5 oder 7, wobei die biologische Probe eine biologische Flüssigkeit ist, die bevorzugt aus Urin, Blut, Plasma oder Serum, bevorzugter Plasma oder Serum ausgewählt ist.

13. Verfahren nach Anspruch 12, wobei wenn die biologische Probe Plasma oder Serum ist, Hämolyse in der biologischen Probe bestimmt wird, und die Probe nicht dem Verfahren unterzogen wird, wenn eine nachweisbare Hämolyse vorhanden ist.

14. Verfahren nach Anspruch 13, wobei eine Hämolyse spektrophotometrisch oder durch Analysieren von Expressionswerten von hämolysebedingten miRNAs, die in hämolysierten Proben hochreguliert sind, oder durch beide bestimmt wird.

15. Verfahren nach Anspruch 14, wobei die hämolysebedingten miRNAs eine Vielzahl der miRNAs miR-451, miR-486-5p, miR-16, miR-92a oder miR-140-3p umfassen, oder wobei die hämolysebedingten miRNAs miR-451, miR-486-5p, miR-16 und miR-92a sind.

16. Verfahren nach Anspruch 14, ferner umfassend das Bestimmen von Expressionswerten einer Vielzahl von normalisierenden miRNAs, die nicht in hämolysierten Proben hochreguliert sind, um die Expressionswerte der hämolysebedingten miRNAs zu normalisieren, wobei wahlweise die normalisierenden miRNAs miR-126, miR-15b, miR-221 und miR-30b umfassen, wobei wahlweise eine Hämolyse ferner bestimmt ist durch
(a) Bestimmen des Expressionsverhältnisses von jedem von 16 miRNA-Paaren, bestehend aus jedem von miR-451, miR-486-5p, miR-16 und miR-92a, gepaart mit jedem von miR-126, miR-15b, miR-221 und miR-30b in der Probe;
(b) Vergleichen von jedem der 16 Expressionsverhältnisse aus Schritt (a) mit einem Schwellenwert, der für jedes Expressionsverhältnis aus dem durchschnittlichen Verhältnis einer Vielzahl von entsprechenden miRNA-Paaren aus einer Vielzahl von Kontrollproben bestimmt wird;
(c) für jedes der 16 miRNA-Paare Zuweisen eines positiven Scores für das Expressionsverhältnis in Schritt (a), wenn das Verhältnis den Schwellenwert in Schritt (b) übersteigt, oder Zuweisen eines nicht positiven Scores für das Expressionsverhältnis in Schritt (a), wenn das Verhältnis den Schwellenwert in Schritt (b) nicht übersteigt; und
(d) Kategorisieren der Probe als (i) eine Hämolyse aufweisend, wenn acht oder mehr von den 16 Verhältnissen den Schwellenwert übersteigen, oder (ii) als keine Hämolyse aufweisend, wenn weniger als acht von den 16 Verhältnissen den Schwellenwert übersteigen.

17. Verfahren nach einem der Ansprüche 1, 3, 5 oder 7, ferner umfassend ein Screening des Subjekts auf Lungenkrebs unter Verwendung eines Systems, das keine Analyse einer miRNA-Expression umfasst, vor der oder gleichzeitig mit der miRNA-Expressionsanalyse, wobei wahlweise das System einen Bluttest, ein Röntgen, eine Computertomographie, Positronen-Emissionstomographie, Pleurapunktion, Bronchoskopie, Feinnadelaspiration, Thorakoskopie, Thorakotomie, Mediastinoskopie, bevorzugt niedrig dosierte Computertomographie (low-dose computed tomography, LDCT), umfasst.

18. Verfahren nach einem der Ansprüche 1, 3, 5 oder 7, wobei das Verfahren an mindestens zwei verschiedenen biologischen Proben vom Subjekt durchgeführt wird, wobei bevorzugt mindestens eine der mindestens zwei verschiedenen biologischen Proben von dem Subjekt entnommen wird, nachdem das Subjekt für Lungenkrebs behandelt worden ist.

19. Verfahren nach einem der Ansprüche 1, 3, 5, 7 oder 9, wobei das Subjekt nicht für Lungenkrebs behandelt wird, wenn ein negatives Vorhandensein oder Risiko bestimmt wird, oder wenn ein niedriges Risiko bestimmt wird; oder das Subjekt für Lungenkrebs behandelt wird, wenn ein positives Vorhandensein oder Risiko bestimmt wird, oder wenn ein intermediäres oder hohes Risiko bestimmt wird.

## Revendications

1. Méthode permettant de déterminer si un sujet présente une tumeur pulmonaire, la méthode comprenant les étapes qui consistent à :
(a) déterminer le rapport d'expression d'une paire de miARN dans un échantillon biologique obtenu chez le sujet ;
(b) comparer le rapport d'expression de l'étape (a) à une valeur seuil déterminée à partir du rapport moyen d'une pluralité de paires de miARN correspondants provenant d'une pluralité d'échantillons témoins ;
(c) assigner un score positif au rapport d'expression de la paire de miARN de l'étape (a) si le rapport dépasse la valeur seuil de l'étape (b) ou assigner un score non positif au rapport d'expression de la paire de miARN de l'étape (a) si le rapport ne dépasse pas la valeur seuil de l'étape (b) ;
(d) répéter les étapes (a) à (c) pour des paires de miARN additionnelles soit (1) jusqu'à ce qu'un score positif soit assigné aux rapports d'expression d'au moins neuf paires de miARN, soit (2) jusqu'à ce qu'un score positif soit assigné aux rapports d'expression de moins de neuf paires de miARN après comparaison des rapports d'expression de 27 paires de miARN,
les paires de miARN comprenant une ou plusieurs de chacune des suivantes : 106a/140-5p, 106a/142-3p, 126/140-5p, 126/142-3p, 133a/142-3p, 140-5p/17, 142-3p/148a, 142-3p/15b, 142-3p/17, 142-3p/21, 142-3p/221, 142-3p/30b et 320/660 ou rapports inverses de celles-ci ; et
(e) catégoriser la présence d'une tumeur pulmonaire comme étant (i) positive si un score positif est assigné aux rapports d'expression d'au moins neuf de paires de miARN ou (ii) négative si un score positif est assigné aux rapports d'expression de moins de neuf paires de miARN.

2. Méthode de la revendication 1, les paires de miARN comprenant en outre une ou plusieurs ou chacune des suivantes : 106a/660, 106a/92a, 126/660, 140-5p/197, 140-5p/28-3p, 142-3p/145, 142-3p/197, 142-3p/28-3p, 17/660, 17/92a, 197/660, 197/92a, 19b/660 et 28-3p/660 ou rapports inverses de celles-ci.

3. Méthode permettant de déterminer si un sujet présente une tumeur pulmonaire agressive, comprenant les étapes qui consistent à :
(a) déterminer le rapport d'expression d'une paire de miARN dans un échantillon biologique obtenu chez le sujet ;
(b) comparer le rapport d'expression de l'étape (a) à une valeur seuil déterminée à partir du rapport moyen d'une pluralité de paires de miARN correspondants provenant d'une pluralité d'échantillons témoins ;
(c) assigner un score positif au rapport d'expression de la paire de miARN de l'étape (a) si le rapport dépasse la valeur seuil de l'étape (b) ou assigner un score non positif au rapport d'expression de la paire de miARN de l'étape (a) si le rapport ne dépasse pas la valeur seuil de l'étape (b) ;
(d) répéter les étapes (a) à (c) pour des paires de miARN additionnelles soit (1) jusqu'à ce qu'un score positif soit assigné aux rapports d'expression d'au moins quatorze paires de miARN, soit (2) jusqu'à ce qu'un score positif soit assigné aux rapports d'expression de moins de quatorze paires de miARN après comparaison des rapports d'expression de 28 paires de miARN,
les paires de miARN comprenant une ou plusieurs de chacune des suivantes : 106a/16, 106/660, 16/17, 16/320, 17/660, 197/30b, 197/30c, 320/451, 320/486-5p et 320/660 ou rapports inverses de celles-ci ; et
(e) catégoriser la présence d'une tumeur pulmonaire agressive comme étant (i) positive si un score positif est assigné aux rapports d'expression d'au moins quatorze paires de miARN ou (ii) négative si un score positif est assigné aux rapports d'expression de moins de quatorze paires de miARN.

4. Méthode de la revendication 3, les paires de miARN comprenant en outre une ou plusieurs de chacune des suivantes : 106a/451, 106a/486-5p, 126/451, 126/486-5p, 126/660, 140-5p/197, 16/197, 17/451, 17/486-5p, 197/451, 197/486-5p, 197/660, 197/92a, 19b/451, 19b/486-5p, 19b/660, 28-3p/451 et 28-3p/486-5p ou rapports inverses de celles-ci.

5. Méthode permettant de déterminer si un sujet est à risque de développer une tumeur pulmonaire, la méthode comprenant les étapes qui consistent à :
(a) déterminer le rapport d'expression d'une paire de miARN dans un échantillon biologique obtenu chez le sujet ;
(b) comparer le rapport d'expression de l'étape (a) à une valeur seuil déterminée à partir du rapport moyen d'une pluralité de paires de miARN correspondants provenant d'une pluralité d'échantillons témoins ;
(c) assigner un score positif au rapport d'expression de la paire de miARN de l'étape (a) si le rapport dépasse la valeur seuil de l'étape (b) ou assigner un score non positif au rapport d'expression de la paire de miARN de l'étape (a) si le rapport ne dépasse pas la valeur seuil de l'étape (b) ;
(d) répéter les étapes (a) à (c) pour des paires de miARN additionnelles soit (1) jusqu'à ce qu'un score positif soit assigné aux rapports d'expression d'au moins dix paires de miARN, soit (2) jusqu'à ce qu'un score positif soit assigné aux rapports d'expression de moins de dix paires de miARN après comparaison des rapports d'expression de 27 paires de miARN,
les paires de miARN comprenant une ou plusieurs de chacune des suivantes : 133a/92a, 15b/21, 15b/30b, 15b/30c, 16/197 et 28-3p/451 ou rapports inverses de celles-ci ; et
(e) catégoriser le risque de développement d'une tumeur pulmonaire comme étant (i) positif si un score positif est assigné aux rapports d'expression d'au moins dix paires de miARN ou (ii) négatif si un score positif est assigné aux rapports d'expression de moins de dix paires de miARN.

6. Méthode de la revendication 5, les paires de miARN comprenant en outre une ou plusieurs de chacune des suivantes : 101/140-3p, 106a/451, 106a/660, 106a/92a, 126/660, 133a/451, 133a/660, 140-3p/660, 142-3p/15b, 15b/451, 15b/660, 17/451, 17/660, 17/92a, 197/19b, 197/451, 197/660, 197/92a, 19b/660, 28-3p/660 et 320/660 ou rapports inverses de celles-ci.

7. Méthode permettant de déterminer si un sujet est à risque de développer une tumeur pulmonaire agressive, la méthode comprenant les étapes qui consistent à :
(a) déterminer le rapport d'expression d'une paire de miARN dans un échantillon biologique obtenu chez le sujet ;
(b) comparer le rapport d'expression de l'étape (a) à une valeur seuil déterminée à partir du rapport moyen d'une pluralité de paires de miARN correspondants provenant d'une pluralité d'échantillons témoins ;
(c) assigner un score positif au rapport d'expression de la paire de miARN de l'étape (a) si le rapport dépasse la valeur seuil de l'étape (b) ou assigner un score non positif au rapport d'expression de la paire de miARN de l'étape (a) si le rapport ne dépasse pas la valeur seuil de l'étape (b) ;
(d) répéter les étapes (a) à (c) pour des paires de miARN additionnelles soit (1) jusqu'à ce qu'un score positif soit assigné aux rapports d'expression d'au moins quatorze paires de miARN, soit (2) jusqu'à ce qu'un score positif soit assigné aux rapports d'expression de moins de quatorze paires de miARN après comparaison des rapports d'expression de 28 paires de miARN,
les paires de miARN comprenant une ou plusieurs de chacune des suivantes : 106a/142-3p, 126/142-3p, 126/21, 126/92a, 142-3p/17, 142-3p/197, 142-3p/28-3p, 197/19b, 197/660 et 28-3p/660 ou rapports inverses de celles-ci ; et
(e) catégoriser le risque de développement d'une tumeur pulmonaire agressive comme étant (i) positif si un score positif est assigné aux rapports d'expression d'au moins quatorze paires de miARN ou (ii) négatif si un score positif est assigné aux rapports d'expression de moins de quatorze paires de miARN.

8. Méthode de la revendication 7, les paires de miARN comprenant en outre une ou plusieurs de chacune des suivantes : 106a/451, 126/451, 145/197, 17/451, 197/21, 197/30b, 197/30c, 197/451, 197/92a, 19b/451, 21/221, 21/28-3p, 28-3p/30b, 28-3p/30c, 28-3p/451, 28-3p/92a, 320/451 et 320/92a ou rapports inverses de celles-ci.

9. Méthode permettant de prévoir si un sujet est à risque de développer ou de présenter une tumeur pulmonaire, comprenant les étapes qui consistent à :
(a) déterminer la présence d'une signature d'une tumeur pulmonaire de la revendication 1 ;
(b) déterminer la présence d'une signature d'une tumeur pulmonaire agressive de la revendication 3 ;
(c) déterminer le risque de développement d'une signature d'une tumeur pulmonaire de la revendication 5 ;
(d) déterminer le risque de développement d'une signature d'une tumeur pulmonaire agressive de la revendication 7 ;
(e) catégoriser le sujet comme étant à bas risque de développer ou de présenter une tumeur pulmonaire si aucune des signatures des étapes (a)-(d) n'est catégorisée comme étant positive ;
(f) catégoriser le sujet comme étant à risque intermédiaire de développer ou de présenter une tumeur pulmonaire si au moins la présence d'une signature d'une tumeur pulmonaire de l'étape (a) est positive ou si le risque de développement d'une signature d'une tumeur pulmonaire de l'étape (c) est positif ; la présence d'une signature d'une tumeur pulmonaire agressive de l'étape (b) et le risque de développement d'une signature d'une tumeur pulmonaire agressive de l'étape (d) étant tous deux négatifs ; et
(g) catégoriser le sujet comme étant à haut risque de développer ou de présenter une tumeur pulmonaire si au moins la présence d'une signature d'une tumeur pulmonaire agressive de l'étape (b) est positive ou le risque de développement d'une signature d'une tumeur pulmonaire agressive de l'étape (d) est positif.

10. Méthode de l'une quelconque des revendications 1, 3, 5 ou 7, dans laquelle la valeur seuil est déterminée comme étant le rapport moyen obtenu avec la pluralité d'échantillons témoins, le rapport moyen obtenu avec la pluralité d'échantillons témoins +/- un écart type ou le rapport médian obtenu avec une pluralité d'échantillons témoins.

11. Méthode de la revendication 1, 3, 5 ou 7, dans laquelle le rapport d'expression de la paire de miARN est déterminé en fabriquant des copies de l'ADNc de chacun des miARN de chaque paire de miARN en utilisant une réaction en chaîne par polymérase après transcription inverse (RT-PCR), préférablement en utilisant une PCR en temps réel et plus préférablement en utilisant des sondes TaqMan.

12. Méthode de l'une quelconque des revendications 1, 3, 5 ou 7, dans laquelle l'échantillon biologique est un liquide biologique préférablement sélectionné parmi l'urine, le sang, le plasma ou le sérum, plus préférablement le plasma ou le sérum.

13. Méthode de la revendication 12, dans laquelle l'hémolyse de l'échantillon biologique est évaluée quand l'échantillon biologique est le plasma ou le sérum et dans laquelle l'échantillon n'est pas soumis à la méthode si une hémolyse détectable est présente.

14. Méthode de la revendication 13, dans laquelle l'hémolyse est évaluée par spectrophotométrie et/ou analyse des niveaux d'expression des miARN associés à une hémolyse, qui font l'objet d'une régulation positive dans des échantillons hémolysés.

15. Méthode de la revendication 14, dans laquelle les miARN associés à une hémolyse comprennent une pluralité des miARN miR-451, miR-486-5p, miR-16, miR-92a ou miR-140-3p, ou dans laquelle les miARN associés à une hémolyse sont miR-451, miR-486-5p, miR-16 et miR-92a.

16. Méthode de la revendication 14, qui comprend en outre la détermination des niveaux d'expression d'une pluralité de miARN de normalisation qui ne font pas l'objet d'une régulation positive dans des échantillons hémolysés pour normaliser les niveaux d'expression des miARN associés à une hémolyse, les miARN de normalisation comprenant éventuellement miR-126, miR-15b, miR-221 et miR-30b et l'hémolyse étant éventuellement en outre évaluée :
(a) en déterminant le rapport d'expression de chacune des 16 paires de miARN consistant en miR-451, miR-486-5p, miR-16 et miR-92a chacun appariés avec miR-126, miR-15b, miR-221 et miR-30b dans l'échantillon ;
(b) en comparant chacun des 16 rapports d'expression de l'étape (a) à une valeur seuil déterminée pour chaque rapport d'expression à partir du rapport moyen d'une pluralité de paires de miARN correspondants provenant d'une pluralité d'échantillons témoins ;
(c) en assignant, à chacune des 16 paires de miARN, un score positif au rapport d'expression de l'étape (a) si le rapport dépasse la valeur seuil de l'étape (b) ou en assignant un score non positif au rapport d'expression de l'étape (a) si le rapport ne dépasse pas la valeur seuil de l'étape (b) ; et
(d) en catégorisant l'échantillon comme étant (i) hémolysé si huit ou plus des 16 rapports dépassent la valeur seuil ou (ii) non hémolysé si moins de huit des 16 rapports dépassent la valeur seuil.

17. Méthode de l'une quelconque des revendications 1, 3, 5 ou 7, qui comprend en outre le dépistage d'un cancer de poumon chez le sujet au moyen d'un système qui ne fait pas intervenir une analyse de l'expression de miARN utilisé avant ou en même temps que l'analyse de l'expression de miARN, le système comprenant éventuellement une analyse de sang, la radiographie aux rayons X, la tomodensitométrie, la tomographie par émission de positrons, une thoracentèse, une bronchoscopie, une ponction à l'aiguille fine, une thoracoscopie, une thoracotomie, une médiastinoscopie, préférablement la tomodensitométrie à faible dose (LDCT).

18. Méthode de l'une quelconque des revendications 1, 3, 5 ou 7, la méthode étant effectuée sur au moins deux échantillons biologiques différents obtenus chez le sujet, au moins un des au moins deux échantillons biologiques différents étant préférablement obtenu chez le sujet après qu'il ait été traité pour un cancer du poumon.

19. Méthode de l'une quelconque des revendications 1, 3, 5, 7 ou 9, dans laquelle le sujet n'est pas traité pour un cancer du poumon s'il est établi que la présence ou le risque est négatif ou que le risque est faible ; ou le sujet est traité pour un cancer du poumon s'il est établi que la présence ou le risque est positif ou que le risque est intermédiaire ou élevé.
